Europäisches Patentamt

European Patent Office

Office européen des brevets

(11) Veröffentlichungsnummer: **0 267 159**
**A2**

(12) # EUROPÄISCHE PATENTANMELDUNG

(21) Anmeldenummer: 87810628.5

(22) Anmeldetag: 02.11.87

(51) Int. Cl.⁴: **C 12 N 15/00**
A 01 H 1/00, C 12 N 1/20,
C 12 N 5/00, A 01 N 63/00

(30) Priorität: 07.11.86 CH 4456/86
16.06.87 CH 2255/87

(43) Veröffentlichungstag der Anmeldung:
11.05.88 Patentblatt 88/19

(84) Benannte Vertragsstaaten:
AT BE CH DE ES FR GB GR IT LI LU NL SE

(71) Anmelder: CIBA-GEIGY AG
Klybeckstrasse 141
CH-4002 Basel (CH)

LUBRIZOL GENETICS, INC.
29400 Lakeland Boulevard
Wickliffe Ohio 44092 (US)

(72) Erfinder: Grimsley, Nigel Harry, Dr.
Lindenberg 2a
CH-4058 Basel (CH)

Hohn, Barbara, Dr.
Talmattweg 11
CH-4103 Bottmingen (CH)

Hohn, Thomas, Dr.
Talmattweg 11
CH-4103 Bottmingen (CH)

Davies, Jeffrey William, Dr.
7 Meadow Road New Costessey
Norwich NR5 0NF Norfolk (GB)

Boulton, Margaret Irene
The Almonos South Green Mattishall
Dereham Norfolk (GB)

(74) Vertreter: Roth, Bernhard M. et al
Patentabteilung der CIBA-Geigy AG Klybeckstrasse 141
CH-4002 Basel (CH)

(54) **Verfahren zur genetischen Modifikation monokotyler Pflanzen.**

(57) Die vorliegende Erfindung betrifft ein neuartige Verfahren zur Einschleusung von genetischem Material in monokotyle Pflanzen oder lebensfähige Teile davon, das dadurch gekennzeichnet ist, dass Transfer-Mikroorganismen, die in der Lage sind, besagtes genetisches Material in monokotyle Pflanzen oder lebensfähige Teile davon einzuschleusen und die das einzuschleusende genetische Material in einer transportierbaren Form enthalten, in Form einer Bakteriensuspension in die meristematischen Gewebe-Regionen der Pflanze oder lebensfähiger Teil davon, inokuliert werden. Dabei kann durch geeignete Wahl des Applikationszeitpunkts im Hinblick auf das Entwicklungsstadium der Empfängerpflanze die Transformationshäufigkeit noch zusätzlich gesteigert werden.

EP 0 267 159 A2

**Beschreibung**

Verfahren zur genetischen Modifikation monokotyler Pflanzen

Die vorliegende Erfindung betrifft ein neuartiges Verfahren zur Einschleusung genetischen Materials in monokotyle Pflanzen oder lebensfähige Teile davon, unter Verwendung von geeigneten Transfer-Mikroorganismen, die Expression des eingeschleusten genetischen Materials in monokotylen Pflanzen oder lebensfähigen Teilen davon sowie die nach diesem Verfahren erhältlichen transgenen pflanzlichen Produkte.

Angesichts des raschen Anstiegs der Weltbevölkerung und dem damit verbundenen höheren Nahrungsmittel- und Rohstoffbedarf gehört die Steigerung des Ertrags von Nutzpflanzen sowie die vermehrte Gewinnung von pflanzlichen Inhaltsstoffen, also der Fortschritt auf dem Gebiet der Nahrungs- und Heilmittel, zu den dringlichsten Aufgaben der biologischen bzw. biotechnologischen Forschung. In diesem Zusmmenhang sind als wesentliche Aspekte beispielsweise zu nennen: eine Erhöhung der Resistenz von Nutzpflanzen gegen ungünstige Bodenverhältnisse oder gegen Krankheiten und Schädlinge, eine gesteigerte Resistenz gegen Pflanzenschutzmittel wie Insektizide, Herbizide, Fungizide und Bakterizide, und eine günstige Veränderung des Nährstoffgehalts oder des Ernteertrags von Pflanzen. Solche wünschenswerten Effekte könnten allgemein durch Induktion oder vermehrte Bildung von Schutzstoffen, wertvollen Proteinen oder Toxinen sowie durch Eingriffe in die regulatorischen Kreisläufe des Pflanzenmetabolismus herbeigeführt werden. Eine entsprechende Beeinflussung des pflanzlichen Erbgutes kann beispielsweise durch Uebertragung neuer Gene in ganze Pflanzen oder Pflanzenzellen erfolgen.

Viele der aus agrarökonomischer Sicht bedeutendsten Kulturpflanzen gehören zur Klasse der Monokotyledonen, wobei besonders die Familie der Gramineae hervorzuheben ist, die unsere wichtigsten Getreidearten umfasst, wie z.B. Weizen, Gerste, Roggen, Hafer, Mais, Reis, Hirse, u.a.m.

Das grösste Problem bei der Anwendung der rekombinanten DNA Technologie bei Pflanzen aus der Gruppe der Monokotyledonen liegt im Fehlen von geeigneten Transfer-Mikroorganismen begründet, mit deren Hilfe für die praktische Anwendung ausreichend hohe Transformations-Frequenzen erzielt werden können und die somit als Hilfsmittel für einen gezielten Eingriff ins pflanzliche Genom Verwendung finden könnten. Agrobacterium tumefaciens beispielsweise, einer der am meisten verwendeten Transfer-Mikroorganismen zur Einschleusung von genetischem Material in Pflanzen, ist zwar in hervorragender Weise für eine genetische Manipulation zahlreicher dikotyler Pflanzen geeignet, bei Vertretern der Monokotyledonen, insbesondere bei monokotylen Kulturpflanzen, konnten aber bisher noch keine entsprechend zufriedenstellenden Ergebnisse erzielt werden. Aus der Klasse der Monokotyledonen sind nämlich bisher nur einige wenige ausgewählte Familien bekannt geworden, die ebenfalls auf eine Infektion mit Agrobacterium tumefaciens ansprechen und damit zumindestens theoretisch einer genetischen Manipulation zugänglich sein könnten. Bei letzteren handelt es sich jedoch aus agrarökonomischer Sicht um unbedeutende Randgruppen, die allenfalls als Modellpflanzen Bedeutung erlangen könnten. [1)DeCleene M, Phytopath. Z, 113: 81-89, 1985; 2)Hernalsteens JP et al., EMBO J, 3: 3039-41, 1984; 3)Hooykaas-Van Slogteren GMS et al., Nature 311: 763-764, 1984; 4)Graves ACF and Goldman SL, J. Bacteriol., 169(4): 1745-1746, 1987].

In jüngster Zeit entwickelte Transformationsverfahren, die auf einer direkten Einschleusung von exogener DNA in pflanzliche Protoplasten beruhen, wie beispielsweise der 'direkte Gentransfer' (5)Hain et al, 1985; 6)Paszkowski et al., 1984; 7)Potrykus et al, 1985 b, c, d; 8)Lörz et al., 1985, 9)Fromm et al,, 1985) oder die Mikroinjektion (10)Steinbiss und Stabel, 1983; 11)Morikawa und Yamada, 1985), müssen insofern als problematisch angesehen werden, als die Regenerierbarkeit ausgehend von pflanzlichen Protoplasten bei einer Vielzahl von Pflanzenspezies, insbesondere aus der Gruppe der Gramineae, zur Zeit noch ein weitgehend ungelöstes Problem darstellt.

Gerade die Familie der Gramineen enthält aber die aus agrarökonomischer Sicht bedeutendsten Kulturpflanzen wie beispielsweise Weizen, Gerste, Roggen, Hafer, Mais, Reis, Hirse u.a.m., die von besonderem wirtschaftlichem Interesse sind, sodass es als eine vordringliche Aufgabe angesehen werden muss Verfahren zu entwickeln, die es ermöglichen, unabhängig von den zuvor genannten Einschränkungen, auch die Vertreter der Gramineen einer direkten genetischen Modifikation zugänglich zu machen.

Diese Aufgabe konnte nun im Rahmen der vorliegenden Erfindung überraschenderweise mit einfachen Massnahmen gelöst werden. Entgegen allen Erwartungen, hat es sich im Verlauf der im Rahmen dieser Erfindung durchgeführten Untersuchungen überraschenderweise gezeigt, dass durch die Anwendung geeigneter Kultur- und Applikationsmethoden jetzt auch Pflanzen aus der Gruppe der Monokotyledonen unter Einsatz bestimmter Transfer-Mikroorganismen, wie beispielsweise von Agrobacterium tumefaciens, zielgerichtet transformiert werden können, dass heisst, dass nunmehr auch wichtige Vertreter aus der Gruppe der Monokotyledonen, insbesondere zur Familie der Gramineen gehörige Kulturpflanzen, einer Infektion durch besagten Transfer-Mikroorganismus zugänglich sind.

Besonders hervorzuheben ist die Erweiterung des Wirtsspektrums von Agrobacterium tumefaciens auf die Gramineen, wodurch auch bei Vertretern aus dieser Familie ein direkter und gezielter Eingriff ins Genom möglich wird.

Die auf diese Weise tranformierten Pflanzen können mittels geeigneter Nachweismethoden identifiziert werden. Als besonders geeignet erwies sich dabei die Verwendung von Virusgenomen pflanzenpathogener Viren, wie beispielsweise von Maize Streak Virus (MSV), mit deren Hilfe sich erfolgreiche Transformationen anhand der auftretenden Krankheitssymptome sehr effizient nachweisen lassen.

Ein Bestandteil der vorliegenden Erfindung betrifft daher ein Verfahren zur Einschleusung von genetischem Material in monokotyle Pflanzen oder lebensfähige Teile davon, das sich dadurch kennzeichnet, dass man einen Transfer-Mikroorganismus, der das genetische Material in einer transportierbaren Form enthält, durch Anwendung geeigneter Kultur-und Applikationsmethoden, die eine Induktion der Virulenz-Genfunktionen des Transfer-Mikroorganismus ermöglichen, für eine Infektion von Monokotyledonen einsetzbar macht und mit diesem monokotyle Pflanzen oder lebensfähige Teile davon infiziert.

Im Rahmen dieses Verfahren werden die Transfer-Mikroorganismus wie beispielsweise Agrobacterium tumefaciens zweckmässigerweise in einem der üblicherweise für die Kultivierung von Mikroorganismen verwendeten Nährmedien bei einer Temperatur zwischen 15" und 40"C über einen Zeitraum von 30 bis 60 Stunden (h) in einer bewegten Flüssigkultur herangezogen. Die bevorzugte Anzuchttemperatur liegt bei 24" bis 29"C. Anschliessend erfolgen ein oder mehrere Subkultivierungsschritte vorzugsweise im gleichen Medium. zweckmässigerweise in einem Verdünnungsverhältnis von 1:20, die sich jeweils über einen Zeitraum von 15 bis 30 h, vorzugsweise von 18 bis 20 h erstrecken. Auch in diesem Fall liegt die Kultivierungstemperatur bei 15° bis 40°C, vorzugsweise bei 24" bis 29°C.

Ein wesentlicher Bestandteil der vorliegenden Erfindung betrifft daher eine weitergehende Differenzierung des Applikationsortes an der Pflanze und damit die gezielte Applikation der transformierenden, Mikroorganismen-haltigen Inokulationslösung an genau definierte Stellen der Pflanze, was zu einer signifikanten Erhöhung der Transformationshäufigkeit der inokulierten Pflanzen führt. Weiterhin kann durch geeignete Wahl des Applikationszeitpunkts im Hinblick auf das Entwicklungsstadium der Empfängerpflanze die Transformationshäufigkeit noch zusätzlich gesteigert werden.

Die vorliegende Erfindung betrifft somit insbesondere ein neuartiges Verfahren zur Einschleusung von genetischem Material in monokotyle Pflanzen oder lebensfähige Teile davon, das dadurch gekennzeichnet ist, dass Transfer-Mikroorganismen, die in der Lage sind, besagtes genetisches Material in monokotyle Pflanzen oder lebensfähige Teile davon einzuschleusen und die das einzuschleusende genetische Material in einer transportierbaren Form enthalten, in Form einer Mikroorganismensuspension in die meristematischen Gewebe-Regionen der Pflanze oder lebensfähiger Teil davon, inokuliert werden.

Um ein klares und einheitliches Verständnis der Beschreibung und der Ansprüche sowie des Umfangs, der den besagten Ansprüchen zukommen soll, zu gewährleisten, werden im Rahmen der vorliegenden Erfindung die folgende Definitionen aufgestellt:

Transfer-Mikroorganismus: Mikroorganismus, der einen Teil seiner DNA in pflanzliches Material überführen kann (beispielsweise Agrobacterium tumefaciens).

T-Replikon: ein Replikon [13)Jacob F et al., 1963], welches mit Hilfe von Genen, die auf diesem Replikon selbst oder auf einem anderen, im selben Mikroorganismus vorhandenen Replikon lokalisiert sind, in seiner Gesamtheit oder als Teilstück in Pflanzenzellen transportiert werden kann (Beispiel: das Ti-Plasmid von Agrobacterium tumefaciens).

T-DNA-Grenz-Sequenzen: ("border sequences") DNS-Sequenzen, die in einer oder mehreren Kopien mit Hilfe mikrobieller Funktionen DNA-Transfer in pflanzliches Material bewirken.

Cargo-DNA: in einen DNA-Vektor artifiziell eingesetzte DNA.

genomische DNA: aus dem Genom eines Organismus abgeleitete DNA

c-DNA: durch Reverse-Transkriptase hergestellte Kopie einer einer mRNA

synthetische DNA: Eine DNA Sequenz, die für ein spezifisches Produkt, Produkte oder eine biologische Funktion kodiert und die auf synthetischem Wege hergestellt wird.

Heterologe(s) Gen(e) oder DNA: Eine DNA Sequenz, die für ein spezifisches Produkt, Produkte oder eine biologische Funktion kodiert und die von einer anderen Spezies stammt als derjenigen, in welche das besagte Gen eingeschleusst wird; besagte DNA Sequenz wird auch als Fremdgen oder Fremd-DNA bezeichnet.

Homologe(s) Gen(e) oder DNA: Eine DNA Sequenz, die für ein spezifisches Produkt, Produkte oder eine biologische Funktion kodiert und die aus der gleichen Spezies stammt, in welche das besagte Gen eingeschleusst wird.

pflanzliche Zellkulturen: Kulturen von pflanzlichen Einheiten wie beispielsweise Protoplasten, Zellkulturzellen, Zellen in Pflanzengeweben, Pollen, Pollenschläuchen, Eizellen, Embryosäcken, Zygoten und Embryonen in unterschiedlichen Entwicklungsstadien.

Pflanze: Jedes photosynthetisch aktive Mitglied aus dem Reich Planta, das durch einen membranumhüllten Nukleus, ein in Form von Chromosomen organisiertes genetisches Material, membranumhüllte cytoplasmatische Organelle und der Fähigkeit zur Durchführung einer Meiose charakterisiert ist.

Pflanzen-Zelle: Strukturwelle und physiologische Einheit der Pflanze, bestehend aus einem Protoplasten und einer Zellwand.

Protoplast: aus Pflanzenzellen oder -geweben isolierte zellwandlose "nackte Pflanzenzelle" mit der Potenz zu einem Zellklon oder einer ganzen Pflanze zu regenerieren.

Pflanzengewebe: Eine Gruppe von Pflanzenzellen, die in Form einer strukturellen und funktionellen Einheit organisiert sind.

Pflanzenorgan: Ein abgegrenzter und deutlich sichtbar differenzierter Teil einer Pflanze, wie beispielsweise Wurzel, Stamm, Blatt oder Embryo.

Vollständig durchtransformierte Pflanzen: Pflanzen, in denen das Genom jeder Zelle im gewünschten Sinne transformiert ist.

Im einzelnen betrifft die vorliegende Erfindung ein verbessertes Verfahren zur Transformation monokotyler Pflanzen mit Hilfe von Agrobacterium-Stämmen, die in der Lage sind, besagte Transformation durchzuführen, welches sich dadurch kennzeichnet, dass die Wahl des Inokulationszeitpunktes im Hinblick auf das Entwicklungsstadium der Empfängerpflanze und des Inokulationsortes im Bereich der Wachstumszonen in der Weise aufeinander abgestimmt werden, dass eine signifikante Erhöhung der erreichbaren Transformationsraten eintritt im Vergleich zu bekannten Verfahren.

Der erfindungsgemäss bevorzugte Zeitpunkt im Hinblick auf das Entwicklungsstadium der Empfängerpflanze für die Applikation der transformierenden Mikroorganismen-haltigen Suspension erstreckt sich über einen Zeitraum, der mit der Entwicklung des pflanzlichen Embryos beginnt und mit dem Erreichen des Blütenstadiums und damit dem Ende der Wachstums- und Entwicklungs-(Differenzierungs)phase der Empfängerpflanze endet.

Für die Anwendung des erfindungsgemässen Verfahrens eignen sich insbesondere Pflanzen, die ein Entwicklungsstadium erreich haben, welches sich zwischen der Samenkeimung und dem Erreichen des 4-Blattstadiums erstreckt.

In einer weiteren Ausführungsform der vorliegenden Erfindung erfolgt die Inokulation der Mikroorganismen-haltigen, transformierenden Inokulationslösung am unreifen, sich entwickelnden Embryo nach der erfolgten Bestäubung und Befruchtung der Eizellen durch den Spermakern, bevorzugterweise jedoch vor der Ausbildung der Samenschale.

Ganz besonders bevorzugt sind 1-3 Tage alte Keimlinge, bei denen der Abstand zwischen dem Skutellarknoten und dem apikalen Koleoptilarspitze 1-2 cm beträgt. Besonders geeignet sind jedoch Pflanzen, die sich in einem Entwicklungsstadium befinden, das eine eindeutige Identifizierung des Koleoptilarknotens ermöglicht.

Die Inokulation der Mikroorganismen-haltigen transformierenden Suspension erfolgt bevorzugterweise in Regionen der Pflanze, die meristematisches Gewebe enthalten. Es handelt sich dabei um teilungs- und stoffwechselaktive Gewebeabschnitte, die in erster Linie totipotente embryonale Zellen enthalten, aus denen sich die Gesamtheit der somatischen Zellen und Gewebe ausdifferenziert und die damit letztlich auch den Ausgangspunkt für die Entwicklung der Keimzellen bilden.

Durch Anwendung des erfindungsgemässen Verfahrens können daher nicht nur transgene Pflanzen erhalten werden, deren somatische Zellen transformiert sind, sondern in erster Linie auch solche Pflanzen, die transformierte Keimzellen enthalten, aus denen sich im Verlaufe der weiteren Zell- und Gewebe-Differenzierung transformierte Eizellen und/oder Pollen entwickeln können.

Nach erfolgter Befruchtung unter Beteiligung transformierter Eizellen und/oder transformierter Pollen erhält man Samen, der transgene Embryonen enthält und der für die Herstellung transgener Pflanzen verwendet werden kann.

Als besonders geeigneter Applikationsort für das Einbringen der transformierenden, mikroorganismenhaltigen Suspension an bereits in Stengel, Wurzel und Blätter differenzierten Pflänzchen, erweist sich der Grenzbereich zwischen Wurzel und Stengel, der sog. Wurzelhals.

Besonders bevorzugt im Rahmen dieser Erfindung ist eine mehrmalige Applikation der transformierenden Mikroorganismen-haltigen Inokulationslösung in die meristematischen Gewebebereiche der Pflanze.

In einer besonderen Ausführungsform der vorliegenden Erfindung erfolgt die Applikation der transformierenden, Mikroorganismenhaltigen Suspension am Keimling, ca. 1-3 Tage nach der erfolgten Samenkeimung. Bevorzugte Applikationsorte sind der Bereich der Koleoptile und der Koleorhiza.

Sehr gute Transformationsergebnisse lassen sich bei Applikation in unmittelbarer Nachbarschaft oder insbesondere bei Applikation direkt in den Koleoptilarknoten erreichen.

Demnach ist eine weitere, besonders bevorzugte Ausführungsform der vorliegenden Erfindung dadurch gekennzeichnet, dass die Applikation der transformierenden, Mikroorganismen-haltigen Inokulationslösung 1-3 Tage nach der erfolgten Keimung in unmittelbarer Nachbarschaft oder direkt in den Koleoptilarknoten des Keimlings erfolgt.

Das Einbringen der transformierenden, Mikroorganismen-haltigen Inokulationslösung in die Pflanze kann auf vielerlei Art und Weise durchgeführt werden, z.B. durch artifizielle Verletzung des epidermalen Gewebes und Einreiben der Mikroorganismen-haltigen, transformierenden Suspension in das verletzte Gewebe oder durch gemeinsame Inkubation des Transfer-Mikroorganismus und der pflanzlichen Protoplasten.

Bevorzugt ist die Injektion der Inokulationslösung unter Verwendung von Injektionsspritzen, mit deren Hilfe eine sehr genau lokalisierte und damit gezielte Applikation an genau definierten Stellen der Pflanze durchführbar ist.

In der Regel verwendet man hierbei Injektionsspritzen mit austauschbaren Injektionskanülen mit einem Querschnitt zwischen 0,1 und 0,5 mm, angepasst an die Bedürfnisse und besonderen Anforderungen der jeweiligen Pflanzenspezies und deren Entwicklungsstadium zum Zeitpunkt der Applikation. Das applizierte Volumen schwankt ebenfalls in Abhängigkeit von der jeweiligen Pflanzenspezies und deren Entwicklungsstadium zwischen 1 und 20 μl. bevorzuvgt ist ein Applikationsvolumen von 5-10 μl.

Selbstverständlich können für die zielgerichtete Applikation der Inokulationslösung in die Pflanze auch andere geeignete Hilfsmittel verwendet werden, wie beispielsweise sehr fein ausgezogene Glaskapillaren, mit deren Hilfe unter Verwendung von Mikromanipulatoren kleinste Applikationsmengen in genau definierte Gewebebereiche der Pflanze (wie z.B. das Meristem) appliziert werden können.

Die Vorgehensweise bei der Applikation der Inokulationslösung an der Pflanze bzw. am Keimling kann ebenfalls variieren, lässt sich jedoch für verschiedene Pflanzenspezies ohne weiteres optimieren. Diese Optimierungsversuche können von jedem Fachmann im Rahmen eines normalen Optimierungsprogrammes gemäss den Richtlinien der vorliegenden Erfindung ohne nennenswerten Aufwand durchgeführt werden.

Für die Effektivität der Transformation ist neben den bereits genannten Parametern auch die Konzentration und Wachstumsphase der inokulierten Transfer-Mikroorganismen von Bedeutung. Die bevorzugte Konzentration liegt in einem Bereich von $10^5$ bis $10^{10}$ Organismen pro ml Inokulationslösung. Besonders bevorzugt ist eine Inokulationskonzentration von $10^7$ bis $10^9$ Organismen/ml.

Im Rahmen dieser Erfindung durchgeführte Verdünnungsexperimente haben gezeigt, dass mit zunehmender Verdünnung der Inokulationslösung eine Abnahme der Transformationshäufigkeit einhergeht. Dabei liegt die Effektivität des Agrobacterium vermittelten DNA Transfers auf monolyte Pflanzen im gleichen Grössenordnungsbereich wie der DNA-Transfer auf dikotyle Wirtspflanzen (Ergebnis-Teil, Punkt D).

Variationsmöglichkeiten im Rahmen des erfindungsgemässen Verfahren betreffen somit beispielsweise die Wahl der Applikationsmethode, der Einstechtiefe in das pflanzliche Gewebe, der Zusammensetzung und Konzentration der Bakteriensuspension sowie die Anzahl der pro Infektion vorgenommenen Inokulation.

In einer weiteren spezifischen Ausführungsform der vorliegenden Erfindung, erfolgt die Applikation der transformierenden, Mikroorganismen-haltigen Lösung nach Dekapitieren der Koleoptilarspitze im Bereich des Koleoptilarknotens unmittelbar in das Koleoptilarknotengewebe. Dabei kann der grösste Teil der Plumula entfernt werden, ohne dass der Keimling in seiner weiteren Entwicklung nachteilig beeinträchtigt wird.

Eine bevorzugte Applikationsmethode umfasst auch in diesem Fall die Verwendung von Injektionsspritzen, wobei dei Einstechtiefe innerhalb bestimmter Grenzen variiert werden kann, in Abhängigkeit von der Entfernung der Dekapitationsstelle vom Koleoptilarknoten. Bevorzugt ist in jedem Fall eine Inokulation unmittelbar in das Koleoptilarknotengewebe.

Die Applikation der Inokulationslösung kann sowohl in die peripheren Gewebeabschnitte, als auch insbesondere in den zentralen Teil des offengelegten Koleoptilarknoten-Gewebes erfolgen, wobei die meristematischen Gewebeabschnitte besonders bevorzugt sind.

Bei der Verwendung unreifer Embryonen kann neben den bereits erwähnten Inokulationstechniken ein Verfahren angewendet werden, bei dem der Embryo zunächst von der Mutterpflanze präparativ entfernt und anschliessend in einem geeigneten Kulturmedium mit dem Transfer-Mikroorganismus in Kontakt gebracht wird (14) Culture and Somatic Cell Genetics of Plants, Vol. 1, ed. IK Vasil, Academic Press, Inc., 1984; 15) Pareddy DR, et al, 1987, Planta, 170: 141-143, 1987).

Als Transfer-Mikroorganismen, die in der Lage sind, genetisches Material auf monokotyle Pflanzen zu übertragen und die in dem erfindungsgemässen Verfahren eingesetzt werden können, kommen in erster Linie Mikroorganismen in Frage, die ein T-Replikon enthalten.

Unter Mikroorganismen, welche ein T-Replikon enthalten, sind insbesondere Bakterien, vorzugsweise Bodenbakterien und von diesen vor allem solche der Gattung Agrobacterium, zu verstehen.

Es versteht sich von selbst, dass im Rahmen des erfindungsgemässen Verfahrens nur Bakterienstämme zu verwenden sind, die unbedenklich sind, das heisst z.B. Bakterienstämme die in der freien Natur nicht lebensfähig sind, bzw. keine ökologischen Probleme verursachen.

Als T-Replikon kommt besonders ein bakterielles Replikon, wie ein Replikon von Agrobacterium, insbesondere von einem Ti- oder Ri-Plasmid eines Agrobacteriums, in Betracht.

Ti-Plasmide besitzen zwei Regionen, die für die Herstellung transformierter Zellen essentiell sind. Bei dikotylen Pflanzen wird eine davon, die Transfer-DNA Region, auf die Pflanze übertragen und führt zur Induktion von Tumoren. Die andere, die virulenzverleihende (vir) Region, ist nur für die Ausbildung, nicht aber für die Aufrechterhaltung der Tumoren essentiell. Die Transfer-DNA Region kann in ihren Dimensionen durch Einbau von Fremd-DNA vergrössert werden, ohne dass die Uebertragungsfähigkeit beeinträchtigt wird. Durch Entfernen der tumorverursachenden Gene, wodurch die transgenen Planzenzellen nicht-tumorös bleiben und durch Einbau eines selektionierbaren Markers, kann das modifizierte Ti-Plasmid als Vektor für den Transfer von genetischem Material in eine geeignete Pflanzenzelle verwendet werden.

Die vir-Region bewirkt den Transfer der T-DNA Region von Agrobacterium auf das Genom der Pflanzenzelle, unabhängig davon, ob die T-DNA-Region und die vir-Region auf demselben Vektor oder auf verschiedenen Vektoren innerhalb derselben Agrobacterium-Zelle vorliegen. Eine vir-Region auf einem Chromosom induziert ebenso den Transfer der T-DNA von einem Vektor in eine Pflanzenzelle.

Bevorzugt wird ein System zur Uebertragung einer T-DNA-Region von einem Agrobacterium in Pflanzenzellen, das dadurch gekennzeichnet ist, dass die vir-Region und die T-DNA-Region auf verschiedenen Vektoren liegen. Ein solches System ist unter dem Begriff "binäres Vektor-System" bekannt und der die T-DNA enthaltende Vektor wird als ein "binärer Vektor" bezeichnet.

Jeder T-DNA enthaltende Vektor, der in Pflanzenzellen übertragbar ist und der eine Detektion der transformierten Zellen erlaubt, ist für die Verwendung im Rahmen dieser Erfindung geeignet.

Pflanzenzellen oder Pflanzen, die gemäss der vorliegenden Erfindung transformiert worden sind, können mit Hilfe eines geeigneten phenotypischen Markers, selektioniert werden. Beispiele solcher phenotypischer Marker, die aber nicht als limitierend aufzufassen sind, beinhalten Antibiotikaresistenz-Marker, wie beispielsweise Kanamycinresistenz- und Hygromycinresistenz-Gene, oder Herbizidresistenz-Marker. Andere phenotypische Marker sind dem Fachmann bekannt und können ebenfalls im Rahmen dieser Erfindung verwendet werden.

Eine bevorzugte Ausführungsform der vorliegenden Erfindung betrifft ein neuartiges, generell anwendbares Verfahren zur genetischen Modifikation von Pflanzen aus der Gruppe der Monokotyledonen durch Einschleusung viraler DNA oder ihrer Aequivalente in ganze Pflanzen, oder lebensfähige Teile davon.

Es ist bereits in einigen Fällen gelungen, ausgewählte DNA-Fragmente in Virus-DNA einzubauen und dann mit dem Virus in einen anderen Organismus einzuschleusen. Während die meisten Pflanzenviren unter natürlichen Bedingungen durch Insekten überträgen werden, welche bei der Nahrungsaufnahme an infizierten und nicht-infizierten Pflanzen fressen und hierdurch eine Neuinfektion von Pflanzen verursachen, ist dieser Weg für eine gezielte und systematische Uebertragung zu aufwendig und zu schwer kontrollierbar. So würden beispielsweise für eine solche Methode unter kontrollierten Bedingungen herangezüchtete Insektenpopulationen benötigt. Ferner wäre insbesondere bei grossen Mengen an Pflanzenmaterial eine planmässige Virusinfektion sehr schwierig zu erreichen.

Die in der Gentechnologie angewendete Methode der mechanischen Inokulation von Blättern mit Viren ist in der Praxis nur begrenzt anwendbar, da klonierte virale DNA nur in manchen Fällen infektiös ist, in vielen anderen Fällen dagegen nicht. Obwohl es möglich ist, gewisse Arten von Virusgenomen in Bakterien zu klonieren und zu studieren, wie beispielsweise Einzelstrang-DNA-Viren, welche durch Klonierung von Doppelstrang-DNA erhalten werden [16)Mullineaux PM et al., (1984)], können doch viele in Bakterien klonierte Viren nicht wieder in die Pflanzen eingeschleust oder zur Infektion von pflanzlichem Material verwendet werden. Damit ist aber auch die Anwendung von Methoden wie der in-vitro-Mutagenese und der rekombinanten DNA Technologie für grundlegende Untersuchungen wie auch für den Einsatz solcher Viren als Träger von ausgewählter Fremd-DNA ausgeschlossen.

Derartige Probleme treten bei Anwendung des nachstehend beschriebenen, erfindungsgemässen Verfahrens nicht auf.

Besonders bevorzugt sind dabei Konstruktionen, die ein oder mehrere virale Replikons oder Teile eines viralen Replikon in einer Weise eingebaut enthalten, die eine Freisetzung und Replikation des viralen Replikon in der pflanzlichen Zelle erlaubt, unabhängig von der chromosomalen DNA.

Diese Anordnung des viralen Replikons ermöglicht damit eine Freisetzung infektiöser viraler DNA aufgrund einer intramolekularen Rekombination, via Transkription, Revers-Transkription oder anderer Methoden der Umlagerung genetischen Materials.

Besonders bevorzugt im Rahmen der vorliegenden Erfindung ist ein T-Replikon, wie beispielsweise ein Ti-Plasmid oder ein Ri-Plasmid eines Agrobacteriums, welches virale DNA, beispielsweise DNA von Maize-Streak Virus (MSV), welche gewünschtenfalls Cargo-DNA eingebaut enthält, in Nachbarschaft zu einer oder mehreren T-DNA-Grenz-Sequenzen enthält, wobei die Entfernung zwischen viraler DNA und T-DNA-Grenz-Sequenz(en) so gewählt ist, dass die Uebertragung der viralen DNA einschliesslich gegebenenfalls vorhandener Cargo-DNA in pflanzliches Material erfolgt.

Als Cargo-DNA können sowohl homologe als auch heterologe Gen(e) oder DNA sowie synthetische Gen(e) oder DNA gemäss der im Rahmen der vorliegenden Erfindung gemachten Definition verwendet werden.

Die kodierende DNA-Sequenz kann ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und/oder synthetischer DNA.

In diesem Fall kann die cDNA aus demselben Gen stammen wie die genomische DNA oder aber, sowohl die cDNA, wie auch die genomische DNA können aus verschiedenen Genen stammen. In jedem Fall können aber, sowohl die genomischen DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die mehr als einem Stamm, einer Varietät oder einer Spezies derselben Gattung angehören, oder aber von Organismen, die mehr als einer Gattung derselben oder einer anderen taxonomischen Einheit (Reich) angehören, abstammen.

Die verschiedenen DNA-Sequenzabschnitte können mit Hilfe an sich bekannter Methoden miteinander zu einer vollständigen kodierenden DNA-Sequenz verknüpft sein. Geeignete Methoden schliessen beispielsweise die in vivo Rekombination von DNA-Sequenzen, die homologe Abschnitte aufweisen sowie die in vitro Verknüpfung von Restriktionsfragmenten mit ein.

Das erfindungsgemässe Verfahren besteht somit im wesentlichen darin, dass man

a) in ein T-Replikon, wie beispielsweise ein Ti-Plasmid oder ein Ri-Plasmid eines Agrobacteriums, virale DNA, beispielsweise DNA von Maize-Streak Virus (MSV), welche gewünschtenfalls Cargo-DNA eingebaut enthält, in Nachbarschaft zu einer oder mehreren T-DNA-Grenz-Sequenzen einsetzt, wobei die Entfernung zwischen viraler DNA und T-DNA-Grenz-Sequenz(en) so gewählt ist, dass die Uebertragung der viralen DNA einschliesslich gegebenenfalls vorhandener Cargo-DNA in pflanzliches Material erfolgt.

b) anschliessend die Aufnahme des Replikons in einen Transfer-Mikroorganismus veranlasst, wobei das Replikon in den Transfer-Mikroorganismus gelangt,

c) mit dem gemäss b) modifizierten Transfer-Mikroorganismus Pflanzen aus der Gruppe der Monokotyledonen oder lebensfähige Teile davon, infiziert.

Dieses Verfahren gewährleistet, dass nach Induzierung der mikrobiellen Funktionen, welche den Transfer der Plasmid-DNA in die Pflanze fördern, auch die eingesetzte Virus-DNA übertragen wird, einschliesslich der gegebenenfalls vorhandenen Cargo-DNA.

Das erfindungsgemässe Verfahren besteht somit im wesentlichen aus folgenden Teilschritten:

a) Isolierung von viraler DNA oder ihren Aequivalenten (s. weiter unten) aus infizierten Pflanzen, beispielsweise solchen der Gattung Zea und Klonierung dieser DNA in Vektoren eines geeigneten Bakteriums, wie beispielsweise Escherichia coli;

b) Aufbau eines Plasmids ( = BaP), welches ein oder mehr als ein virales Genom oder aber Teile virale Genome enthält, welche sich in Nachbarschaft zu einer oder mehreren T-DNA-Grenz-Sequenze befinden, wobei die Entfernung zwischen viraler DNA und T-DNA-Grenz-Sequenz(en) so gewählt ist, dass die Uebertragung der viralen DNA einschliesslich der gegebenenfalls darin eingebauten Cargo-DNA in ganze Pflanzen oder lebensfähige Teile davon erfolgt;

c) Konstruktion eines Vektor-Systems durch Ueberführung des Plasmids BaP in einen Transfer-Mikroorganismus (beispielsweise Agrobacterium tumefaciens oder Agrobacterium rhizogenes);

d) Infektion ganzer monokotyler Pflanzen oder lebensfähiger Teile davon mit dem vorstehend unter c) aufgeführten Vektor-System.

Die Verwendung von Vektor-Systemen, wie sie vorstehend unter c) beschrieben sind, sowie neue Vektor-Systeme, wie beispielsweise Bakterien des Stammes Agrobacterium tumefaciens(Rif[R])C58(pTi C58; pEAP 200) sowie Agrobacterium tumefaciens C58 (pTiC58; pEAP37), C58 (pTiC58; pEAP29), C58 (pTiC58; pEAP40) sowie C58(pTiC58, MSV 109) zur gezielten Transformation monokotyler Pflanzen oder lebensfähiger Teile davon bilden weitere Gegenstände der vorliegenden Erfindung.

Ganz besonders bevorzugt sind Bakterien des Stammes Agrobacterium tumefaciens C58 (pTiC58; pEAP37), C58 (pTiC58; pEAP29), C58 (pTic58; pEAP40) sowie C58 (pTiC58, MSV 109).

Unter viraler DNA und ihren Aequivalenten sind im Rahmen der vorliegenden Erfindung insbesondere folgende DNA-Typen zu verstehen:

- doppelsträngige DNA-Formen von Einzelstrang-DNA-Viren (beispielsweise Gemini-Viren, wie Maize Streak Virus (MSV))

- natürliche Virus-DNA (beispielsweise CaMV);

- cDNA-Kopien von Virus-RNA oder Viroid-RNA (beispielsweise von Tabak-Mosaik-Virus oder Cadang-Cadang-Viroid);

- jegliche lethalen oder lebensfähigen Mutanten von Viren;

- klonierte DNA unter dem Einfluss viraler Replikations- und/oder Expressionssignale;

- klonierte DNA unter dem Einfluss eukaryontischer Replikations-und/oder Expressionssignale;

- Teile von Virus-DNA;

- Aequivalente der vorstehend aufgeführten DNA-Typen in Tandem-Form und

- Aequivalente der vorstehend aufgeführten DNA-Typen mit eingebauter Cargo-DNA.

Die am Beispiel des vorstehend beschriebenen Vektor-Systems beschriebene Anwendung des erfindungsgemässen Verfahrens hat z.B. folgende gravierende Vorteile:

- Eine Erweiterung des Wirtsbereichs von normalerweise Dikotyledonen-spezifischen Transfer-Mikroorganismen, wie zum Beispiel Agrobacterium tumefaciens oder Agrobacterium rhizogenes, auf Monokotyledonen.

- Die Möglichkeit der systemischen Infektion ganzer Pflanzen durch Verwendung viraler DNA bzw. deren Aequivalente.

- Die Erzielung der Infektiosität von Viren, welche bisher artificziell nicht zur Infektion gebracht werden konnten (beispielsweise Maize-Streak-Virus), unter Umgehung der natürlichen Vektoren wie beispielsweise Insekten.

- Die Möglichkeit der Manipulation von Virus-DNA in einem bakteriellen System wie beispielsweise E. coli.

- Eine Erweiterung des Wirtsbereiches von Viren.

- Eine Vereinfachung der Inokulation durch Vermeidung der DNA-Reinigung und drastische Verminderung der zur Inokulation erforderlichen Menge an Inokulum.

- Möglichkeit der Transformation von Zellen, Geweben und ganzen Pflanzen, wodurch Einschränkungen, die auf Grund von Schwierigkeiten bei der Regeneration ganzer Pflanzen aus Protoplasten auftreten können, überwunden werden.

- Unter Kontrolle bakteriell kodierter Funktionen kann die T-DNA, einschliesslich der ausgewählten viralen DNA, in das Wirtsgenom eingebaut werden. Da in manchen Fällen nach einer Transformation mit Bakterien aus einer Einzelzelle eine ganze Pflanze regeneriert werden kann, kann virale DNA in das nukleare Genom sämtlicher Zellen einer Pflanze eingeführt werden. Solche integrierten Virusgenome können.

a) auf sexuellem Wege auf die Nachkommenschaft übertragen werden;

b) eine Infektion mit überinfizierenden Viren verhindern; und

c) gegebenenfalls als Quelle dienen für weitere, gegebenenfalls auch ausgewählte Cargo-DNA enthaltende Viruskopien, welche sich via Transkription, Revers-Transkription, homologe Rekombination oder andere Methoden der Umlagerung genetischen Materials von der integrierten Kopie absetzen.

d) Ferner kann möglicherweise eine Zweitinfektion ("Superinfection") von pflanzlichem Material, welches Teile viraler Genome in die nukleare DNA eingebaut enthält,

α) zur Entwicklung besserer viralerr Vektoren führen, da die Expression viraler Gene aus der nuklearen DNA vielleicht die Möglichkeit bietet, in dem die Zweitinfektion bewirkenden Virus virale DNA durch Fremd-DNA zu ersetzen; und

β) zum besseren Verständnis der Wirt-Parasiten-Beziehungen und damit zur besseren Schützbarkeit der Pflanzen in erheblichem Masse beitragen.

Es werden somit eine Vielzahl von Ausführungsformen von dem breiten Konzept dieser Erfindung umfasst.

Unter Anwendung des vorstehend beschriebenen Verfahrens lässt sich in das Virusgenom eingebaute Cargo-DNA ebenfalls in pflanzliches Material transportieren und darin ausbreiten. Die Ausbreitung der Viren und damit auch des durch sie transportierten Fremdgens in Pflanzen ist insbesondere dann ausserordentlich vorteilhaft, wenn die Pflanzen asexuell vermehrt werden sollen oder auf direktem Wege und in kürzester Zeit gegen schädigende Einflüsse geschützt werden sollen; beispielsweise durch Einbringen eines Resistenzgens in die Pflanzen.

Das erfindungsgemässe Verfahren ist insbesondere dazu geeignet, ausgewählte Gene und damit eine gewünschte Eigenschaft in pflanzliches Material, aber auch ausgewachsene Pflanzen, einzuschleusen und darin zu verbreiten.

Das erfindungsgemässe Verfahren lässt sich auch im Pflanzenschutz einsetzen, um Pflanzen durch einen Transfer-Mikroorganismus, wie vorstehend beschrieben, gegen Virusbefall zu "immunisieren", indem man die Pflanzen mit einem geschwächten, nicht pathogenen oder nur schwach pathogenen Virus transformiert, was dazu führt, dass man sie auf diese Weise vor unerwünschten, weiteren Virusinfektionen schützt.

Als virale DNA, welche im Rahmen des erfindungsgemässen Verfahrens verwendet werden kann, lässt sich, ohne dass dies eine Beschränkung darstellt, beispielsweise DNA von Caulimoviren, einschliesslich Cauliflower Mosaik Virus (CaMV), des weiteren DNA von Vertretern der Geminiviren, wie z.B. von Bean Golden Mosaic Virus (BGMV), Chloris Striate Mosaic Virus (CSMV), Cassava Latent Virus (CLV), Curly Top Virus (CTV), Maize Streak Virus (MSV), Tomato Golden Mosaic Virus (TGMV) und Wheat Dwarf Virus (WDV) verwenden.

Vertreter aus der Gruppe der Caulimoviren, insbesondere aber Cauliflower Mosaik Viren, sind für eine Verwendung im Rahmen des erfindungsgemässen Verfahrens besonders geeignet, da sie aufgrund ihres Genomaufbaus (doppelsträngige DNA) einer genetischen Manipulation unmittelbar zugänglich sind.

Alle bisherigen Experimente und die damit verbundenen Beobachtungen sprechen dafür, dass auch die Vertreter der Geminiviren, deren Genom aus einzelsträngiger (ss) DNA aufgebaut ist, als Vektoren für die Uebertragung genetischen Materials eingesetzt werden können. Zudem ist auch bekannt, dass im Verlauf des Entwicklungszyklus der Geminiviren doppelsträngige ds-DNA in infizierten Pflanzen gebildet wird und dass diese ds-DNA infektiös ist [17)Kegami M et al., Proc. Natl. Acad. Sci., USA, 78: 4102, 1981].

Damit kommen auch Vertreter aus der Gruppe der Geminiviren, die im Verlaufe ihres Entwicklungszyklus ds-DNA bilden und damit einer direkten genetischen Manipulation zugänglich sind, im Rahmen der vorliegenden Erfindung als Träger fremden genetischen Materials in Frage.

Ein weiterer Gegenstand der vorliegenden Erfindung betrifft die Verwendung von Geminiviren als Marker, da sich erfolgreiche Genübertragungen auf Pflanzen unter Einsatz von Viren sehr leicht an den meist makroskopisch sichtbaren Infektionssymptomen erkennen lassen, z.B. an Hand von gelben Punkten oder Streifen, an der Basis neugebildeter Blätter bei Verwendung von MSV.

Der Wirtsbereich der Geminiviren umfasst eine ganze Reihe von wirtschaftlich bedeutenden Kulturpflanzen, wie Mais, Weizen, Tabak, Tomaten, Bohnen, sowie zahlreiche tropische Pflanzen.

Von besonderer kommerzieller Bedeutung ist der Wirtsbereich von Maize Streak Virus, der zahlreiche monokotyle Kultur- und Getreidepflanzen umfasst, wie z.B. Mais, Reis, Weizen, Hirse, Sorghum, sowie verschiedene afrikanische Gräser.

Das erfindungsgemässe Verfahren ist insbesondere geeignet zur Infektion ganzer Pflanzen aus der Klasse der Monocotyledoneae oder lebensfähiger Teile dieser Pflanzen, wie z.B. pflanzliche Gewebekulturen oder Zellkulturzellen mit viraler DNA sowie deren Aequivalenten. Ein weiterer Gegenstand dieser Erfindung betrifft daher die aus dem erfindungsgemässen Verfahren resultierenden transformierten Protoplasten, Pflanzenzellen, Zellklone, Zellaggregate, Pflanzen und Samen sowie deren Nachkommen, die die durch die Transformation erzeugte neue Eigenschaft aufweisen, ferner alle Kreuzungs- und Fusionsprodukte des transformierten Pflanzenmaterials, die die durch die Transformation erzeugten neuen Eigenschaften aufweisen.

Ebenso ein Bestandteil der vorliegenden Erfindung sind transformierte ganze Pflanzen sowie lebensfähige Teile dieser Pflanzen, insbesondere Pollen, Eizellen, Zygoten, Embryonen oder jedes beliebige andere aus transformierten Keimbahnzellen hervorgegangene Vermehrungsgut.

Weiterhin werden durch die vorliegende Erfindung auch vollständig durchtransformierte Pflanzen umfasst, die aus lebensfähigen Teilen transformierter monokotyler Pflanzen regeneriert worden sind.

Monokotyle Pflanzen, die für die erfindungsgemässe Verwendung geeignet sind, schliessen beispielsweise Spezies aus den Familien Alliaceae, Amaryllidaceae, Asparagaceae, Bromeliaceae, Gramineae, Liliaceae, Musaceae, Orchidaceae oder Palmae ein.

Besonders bevorzugt sind dabei Vertreter aus der Familie der Gramineae, wie z.B. Pflanzen die grossflächig angebaut werden und hohe Ernteerträge liefern. Als Beispiele seien genannt: Mais, Reis, Weizen, Gerste, Roggen, Hafer oder Hirse.

Weitere Zielkulturen für die Anwendung des erfindungsgemässen Verfahrens sind beispielsweise Pflanzen der Gattungen Allium, Avena, Hordeum, Oryzae, Panicum, Saccharum, Secale, Setaria, Sorghum, Triticum, Zea, Musa, Cocos, Phoenix und Elaeis.

Der Nachweis einer erfolgreichen Transformation durch Uebertragung von MSV DNA auf die jeweilige Testpflanze kann auf an sich bekannte Weise erfolgen, beispielsweise anhand von Krankheitssymptomen sowie durch molekularbiologische Untersuchungen, zu denen besonders die "Southern-blot"-Analyse gehört.

Die extrahierte DNA wird zunächst mit Restriktionsenzymen behandelt, anschliessend einer Elektrophorese in 1 %-igem Agarose-Gel unterworfen, auf eine Nitrozellulosemembran [22) Southern, E.M., J.Mol.Biol. 98, 503-517 (1975)] transferiert und mit der nachzuweisenden DNA, welche zuvor einer Nick Translation [23) Rigby, W.J., Dieckmann, M., Rhodes, C. and P. Berg, J.Mol.Biol. 113, 237-251)] unterworfen wurde (DNA-spezifische Aktivitäten von 5 x 10$^8$ bis 10 x 10$^8$ c.p.m./μg) hybridisiert. Die Filter werden dreimal je eine Stunde lang mit einer wässrigen Lösung von 0.03 M Natriumcitrat und 0.3 M Natriumchlorid bei 65°C gewaschen. Die hybridisierte DNA wird durch Schwärzung eines Röntgenfilms während 24 bis 48 Stunden sichtbar gemacht.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn es wird spezielle darauf hingewiesen.

Nichtlimitierende Ausführungsbeispiele:

Beispiel 1: Konstruktion eines Vektors mit dimerem MSV-Genom

Die Isolierung von MSV-Genom kann aus in der Natur vorhandenen, infizierten Maispflanzen gemäss [16]Mullineaux PM et al., EMBO J, 3: 3063-3068, 1984 erfolgen. Dabei dient Virion ss DNA als Matritze für die in vitro Synthese doppelsträngiger MSV-DNA unter Verwendung von Klenow-Polymerase I und einem endogenen Primer [18]Donson J et al., EMBO J, 3: 3069-3073, 1984].

Eine andere Möglichkeit besteht in der Isolierung von doppelstängiger MSV-DNA ('supercoiled MSV-DNA') direkt aus infiziertem Blattmaterial. Doppelsträngige MSV-DNA wird als Intermediärprodukt bei der Virus-Replikation gebildet. Sie wird als 'replicative form DNA' oder 'RF-DNA' bezeichnet.

Die Klonierung der MSV-Genome erfolgt durch Einbau der RF-DNA bzw. der in vitro synthetisierten DNA in einen durch BamHI linearisierten pUC9 Vektor [19] Vieira T and Messing T, Gene 19: 259 - 268, 1982]. Zur Identifizierung der rekombinanten Phagen wird der lac Komplementations-Test herangezogen.

Die weitere Vorgehensweise besteht zunächst im Ausschneiden der klonierten MSV-DNA an der einzigen BamHI-Restriktionsschnittstelle. Das anfallende linearisierte DNA-Fragment wird anschliessend nach gelelektrophoretischer Auftrennung des DNA-Gemisches, isoliert. [20]Maniatis et al., (1982)].

Bei Virusstämmen mit zwei oder mehreren BamHI-Restriktionsschnittstellen wird das MSV-Genom entweder partiell verdaut oder man sucht eine andere geeignete Schnittstelle, die nur einmal im MSV-Genom vorkommt. Letzteres gilt auch für den Fall, dass keine BamHI Schnittstelle im MSV-Genom existiert.

Es folgt das Einspleissen des BamHI-Fragmentes in Tandem-Anordnung in die BglII Schnittstelle des Plasmids pGA471 [21]An G et al., EMBO J, 4: 277-284, 1985], welche sich zwischen den T-DNA-Grenzsequenzen befindet. Dieses sogenannte Tandem-Klonieren kann über die jeweiligen Konzentrationen von Vektor und Insert gesteuert werden. In der Ligationslösung sollte das Insert im Ueberschuss vorliegen. Das bevorzugte Konzentrationsverhältnis liegt in diesem Fall bei 10:1 (Insert:Vektor).

Bei dem Plasmid pGA471 handelt es sich um einen sogenannten Shuttle-Vektor, der sowohl in E.coli wie auch in Agrobacterium tumefaciens in Anwesenheit von Tetrazyklin stabil repliziert wird.

Dieser Vektor besitzt neben dem zwischem den T-DNA Grenzsequenzen liegenden ColEI-Replikationsursprung einen weiteren Replikationsursprung, der einen weiten Wirtsbereich umfasst und die Erhaltung des Plasmids in Agrobacterium tumefaciens erlaubt. Dieser Replikationsursprung stammt aus dem Plasmid pTJS75, einem Plasmid mit weitem Wirtsbereich und einem Tetrazyklin-Resistenz-Gen, einem Abkömmling von RK2 [21]An G et al., EMBO -J, 4: 277-284, 1985).

Weitere charakteristische Eigenschaften des Plasmids pGA471 sind:
1) Der Besitz verschiedener Restriktionsschnittstellen zwischen den T-DNA-Grenzsequenzen, die einen Einbau von Fremd-DNA ermöglichen; 2) eine cos-Region des Bakteriophagen λ, die eine Klonierung grosser DNA-Fragmente (25-35 kb) erlaubt; 3) ein chimäres Markergen, zusammengesetzt aus den Kontrollsequenzen des Nopalin-Synthase-Gens (nos) und einer für Neomycin-Phosphotransferase codierenden DNA-Sequenz sowie 4) eine bom-Schnittstelle, die eine Mobilisierung des Plasmids aus E. coli in Agrobacterium tumefaciens ermöglicht.

Die oben erwähnte Inkubationslösung, enthaltend den Vektor sowie die einzuspleissende MSV-DNA, bevorzugterweise in einem Konzentrationsverhältniss von 1:10, wird für die Transformation des E.coli Stammes DH1 [22]Hanahan D and Meselson M, Gene,10: 63-67, 1980] verwendet. Die Selektionierung erfolgt aufgrund der Tetrazyklin-Resistenz der transformierten Klone sowie von Hybridisierungsexperimenten unter Verwendung radioaktiv markierter MSV-DNA.

Eine Auswahl positiver Klone wird anschliessend auf das Vorhandensein von MSV-Genomen in Tandem-Anordnung untersucht. Dazu wird die Plasmid-DNA nach an sich bekannten Methoden [20]Maniatis et al., (1982)] aus den positiven Klonen isoliert und anschliessend einer Restriktionsanalyse unterzogen.

Einer der 'Tandem Klone' wird selektioniert und von E.coli DH1 auf Agrobacterium tumefaciens (RifR) C58 (pTiC58) übertragen.

Die Uebertragung erfolgt in einem 'triparenteral mating', wie bei [23]Rogers SG et al. (1986) ausführlich beschrieben. Zur Selektion werden in diesem Fall Rifampicin (100 μg/ml) und Tetracylin (5μg/ml) verwendet. Die erfolgreiche Uebertragung der dimeren MSV-Genome wird mit Hilfe der Southern Hybridisierung getestet [24]Dhaese P et al., Nucleic Acids Res. 7: 1837-1849, 1979].

Agrobacterium tumefaciens (RifR) C58 (pTiC58) [25]Holsters et al., Plasmid, 3, 212, 1980], enthält ein Wildtyp Ti-Plasmid mit intakten Virulenzfunktionen, wodurch die Mobilisierung des 'Shuttle Vektors' in die pflanzliche Zelle ermöglicht wird.

Der auf die oben beschriebene Weise transformierte Agrobacterium-Stamm erhält folgende Stammbezeichnung: Agrobacterium tumefaciens (RifR) C58 (pTiC58; pEAP 200).

## Beispiel 2: Konstruktion eines Kontrollvektors

Zur Konstruktion eines Kontrollvektors ohne T-DNA Grenzsequenzen wird das Plasmid pRK252 KanIII verwendet, ein Abkömmling des Plasmids pRK [[26]Bevan M, Nucleic Acid Res., 12: 204-207, 1984)], das keine T-DNA Grenzsequenzen aufweist.

Der Einbau des dimeren MSV-Genoms in den Kontrollvektor erfolgt durch Einspleissen des oben beschriebenen BamHI Fragmentes (siehe Seite 24) in Tandem-Anordnung mit Hilfe eines SalI/BamHI Adaptors in die SalI Schnittstelle des Plasmids pRK252 KanIII.

Die Mobilisierung des Kontrollvektors in Agrobacterium tumefaciens (RifR) C58 (pTiC58) erfolgt über ein 'triparenteral mating', wie bei [23]Rogers SG et al., 1986) ausführlich beschrieben.

Der transformierte Agrobacterium-Stamm erhält folgende Stammbezeichnung: Agrobacterium tumefaciens (RifR) C58 (pTiC58; pEA 21).

## Beispiel 3: Herstellung des bakteriellen Vektors pEAP 25

Durch Austausch eines 0,65 kb Hind III-Sal I Fragments im Cosmid pHC 79, einem Derivat des E.coli Plasmids pBR322 ([27]Hohn and Collins, 1980), durch ein 1,2 kb umfassendes Fragment aus dem Transposon Tn 903 ([28]Grindley et al., 1980), welches ein Kanamycin-Resistenzgen trägt, entsteht das hybride Cosmid p22G1. Die Integration des 1,2 kb Fragments in die Hind III-Sal I Schnittstelle von pHC 79 wird ermöglicht durch Anfügen von Hind III-Sal I Linker-Sequenzen.

Aus dem Plasmid pCaMV6Km wird ein 2,9 kb umfassendes Sal I-Bst EII Fragment, welches ein Gen enthält, das für Kanamycinresistenz bei Pflanzen kodiert ([6]Paszkowski et al., 1984), ausgeschnitten und mit einem 2,4 kb Sal I-Bst EII Fragment aus p22G1 ausgetauscht.

Die endgültige Konstruktion von pEAP 25 erfolgt durch Integration des zuvor mit Sal I geschnittenen Plasmids pB6 in die Sal I Schnittstelle von pEAP 1. Das Plasmid pB6 wurde von J. Davies vom John Innes Institut, Norwich, England entwickelt und zur Verfügung gestellt. Dieses Plasmid ist inzwischen bei [29] N. Grimsley et al., 1987 unter Bezeichnung pMSV 12 publiziert.

Das Plasmid pB6 enthält ein dimeres MSV-Genom, welches zuvor in dem Plasmid pACYC184 ([30]Chang and Cohen, 1978) kloniert wurde.

## Beispiel 4: Herstellung des bakteriellen Vektors pEAP 37

Der bakterielle Vektor pEAP 37 wird konstruiert durch Einfügen des Plasmids pB6, das zuvor mit Sal I geschnitten wurde, in die Sal I Restriktionsschnittstelle des Plasmids pCIB 10. Das Plasmid pCIB 10 wurde von Mary-Dell Chilton, CIBA-GEIGY Biotechnology Facility, Research Triangle Park, Raleigh N.C., U.S.A., entwickelt und zur Verfügung gestellt.

## Beispiel 5: Herstellung des bakteriellen Vektors pEAP 40

Ein 1,6 mer des MSV Genoms [BglII-BamHI-Fragment (0,6 mer) + BamHI-BamHI-Fragment, (Monomer)] wird in die BamHI Restriktionsstellen des Plasmids pTZ19R, das bei [31]Mead et al. (1986) beschrieben ist, eingespleisst. Das so entstandene Plasmid mit der Bezeichnung p3547, das ein 1,6 mer des MSV Genoms enthält, wird mit EcoRI geschnitten und anschliessend in die EcoRI-Stelle des Plasmids pCIB200 ([32]Rothstein et al., 1987) eingespleisst. Durch diese Massnahmen werden die MSV-Sequenzen zwischen die T-DNA "border"-Sequenzen von pCIB200 plaziert.

## Beispiel 6: Konstruktion des bakteriellen Vektors pMSV 109

5 µg des Plasmids pMSV12, dessen Konstruktion zuvor im Beispiel 3 beschrieben wurde, werden mit BamHI in einer Pufferlösung ([20]Maniatis et al., 1982) für einen Zeitraum von 2 Stunden bei einer Temperatur von 37°C verdaut. Das aus dieser enzymatischen Verdauung resultierende 2,7 Kb umfassende DNA-Fragment wird nach der elektrophoretischen Auftrennung der Probe in einem 1 % Agarose-TAE Gel (40 mM Tris-HCl, 20 mM Natriumacetat, 2 mM EDTA) aus diesem eluiert und in die einzige BamHI-Schnittstelle des binären T-DNA-Vektors pBin19 ([26]Bevan, 1984) eingespleisst.

Für die Verküpfungsreaktion arbeitet man mit einem 100fachen molaren Ueberschuss des 2.7 Kb MSV-Fragments (des "Inserts") im Vergleich zum Vektor pBin19 und einer hohen T4-DNA Ligase Konzentration, um eine hohe Einbaurate der dimeren MSV-DNA in den Vektor zu gewährleisten. Die im einzelnen verwendeten Konzentrationen liegen bei 625 ng pMSV DNA und 25 ng pBin19 DNA, die bei einer Temperatur von 10°C für einen Zeitraum von 16 Stunden in Gegenwart von 5 Einheiten T4-DNA Ligase in einem Gesamtvolumen von 10 µl miteinander verknüpft werden. Eine Hälfte dieser Ligations-Mischung wird in kompetente E.coli JM83 recA Zellen transformiert und auf "Luria Broth" (LB)-Agar ([20]Maniatis et al. 1982), der mit 50 µg/ml Kanamycin-Sulfat und 40 µg/ml 5-Dibrom-4-chlor-3-indolylgalactosid (X-gal) angereichert ist, ausplattiert und bei 37°C über Nacht inkubiert.

Weisse Kolonien, die das MSV-Insert enthalten, werden ausgelesen und ein Klon, der das dimere MSV-Insert in Tandem-Anordnung enthält (pMSV109) wird für die Konjugation in Agrobacterium tumefaciens C58NalR ([33]Hepburn et al., 1985) ausgewählt, die nach einem Verfahren, das bei Ditta et al., 1980 beschrieben ist, durchgeführt wird. Die Selektion der Exkonjuganten erfolgt auf LB-Agar enthaltend 50 µg/ml Kanamycinsulfat und 50 µg/ml Nalidixinsäure. Die gewählte Kolonie, die in den im folgenden beschriebenen Inokulationsexperimenten eine Infektion bei Mais auslöst, erhält die Bezeichnung pMSV 114.

Beispiel 7: Herstellung eines Kontrollvektors (pEA 2) ohne T-DNA-Grenzsequenzen

Zur Konstruktion des Kontroll-Vektors pEA 2 wird die Sal I Schnittstelle des Plasmids pRK 252/kmIII, eines Vorläufer-Plasmids von pBIN 19 (26)Bevan, 1984) mit dem Sal I geschnittenen Plasmid pB6 verknüpft. Die Selektionierung von pEA 2 erfolgt aufgrund der Kanamycin (KmR) und Chloramphenicol (CmR)-Resistenz des Kontroll-Vektors.

Beispiel 8: Einführung der Plasmide pEAP 25, pEAP 37 und pEA 2 in Agrobacterium tumefaciens

Das Plasmid pEAP 25 wird in Bakterien des Stammes Escherichia coli GJ23 (pGJ28, R64rd11) (33)van Haute et al., 1983) kloniert. Dieser E.coli-Stamm ermöglicht den konjugalen Transfer von Plasmiden, welche eine bom-Schnittstelle aufweisen, in Agrobacterium tumefaciens. Die Plasmide pEA 2, pEAP 37 und pEAP 40 werden via eines "triparenteral mating" in Agrobacterium tumefaciens mobilisiert (23)Rogers, S.G. et al., 1986). Als Empfängerstämme werden zwei Agrobacterium tumefaciens-Stämme verwendet

1) C58 (pTiC58) für die binären Vektoren pEAP 40, pEA 2 und pEAP 37

2) C58 (pTiC58, pEAP 18) für das Plasmid pEAP 25.

Wildtyp-Stämme von Agrobacterium tumefaciens können von der "Culture Collection of the Laboratory of Microbiology, Microbiology Department of the University of Gent" bezogen werden.

pEAP 25: Als Rezipienten-Stamm für das Plasmid pEAP 25 dient der Agrobacterium-Stamm (pTiC58, pEAP 18). Bei pEAP 18 handelt es sich um einen binären Vektor, welcher konstruiert wird, indem man das 6,7 kb umfassende EcoRI-BamHI-Fragment des Plasmids pGA472 (21)An, G. et al., 1985) durch das aus 2,6 kb bestehende EcoRI-BglII-Fragment des Plasmids pHC79 (27) Hohn, B. et al., 1980), welches zwischen T-DNS-Grenz-Sequenzen einen Bereich für homologe Rekombination in dem Plasmid pEAP 25 enthält, ersetzt. Da das Plasmid pEAP 25 in Agrobacterium tumefaciens nicht zur Replikation gelangt, ergibt die Selektionierung der Exkonjuganten auf Rifampicin, Kanamycin und Carbenicillin den neuen Agbrobacterium-Stamm Agrobacterium tumefaciens C58 (pTic58, pEAP 29), worin das Plasmid pEAP 25 in den binären Vektor pEAP 18 durch homologe Rekombination integriert worden ist.

pEAP 37, pEAP 40: Die Mobilisierung der Plasmide pEAP 37 und pEAP 40 aus E.coli in Agrobacterium tumefaciens via "triparenteral mating" führt zum Aufbau eines binären Vektor-Systems.

pEA 2: Das Kontroll-Plasmid pEA 2 wird in den Agrobacterium-Stamm C58 (pTiC58) eingeschleust, wo es sich in trans zu dem dort bereits vorhandenen Ti-Plasmid etabliert.

Die, wie vorstehend beschrieben, neukonstruierten Plasmide werden anhand von DNA-Isolierung und Restriktionskartierung geprüft.

Die im Rahmen der vorliegenden Erfindung verwendeten Plasmide pEAP 37, pEAP 40 und pMSV 109 wurden bei der als Internationale Hinterlegungsstelle anerkannten 'Deutschen Sammlung von Mikroorganismen (DSM), in Göttingen, Bundesrepublik Deutschland, bzw. 'The National Collection of Industrial Bacteria' (NCIB), Torry Research Station, P.O. Box 31, 135 Abbey Road, Aberdeen entsprechend den Anforderungen des Budapester Vertrages für die Internationale Anerkennung der Hinterlegung von Mikroorganismen zum Zwecke der Patentierung, hinterlegt. Eine Erklärung zur Lebensfähigkeit der hinterlegten Proben wurde durch die besagte Internationale Hinterlegungsstelle ausgefertigt.

0 267 159

| Mikroorganismen | Hinterlegungs-datum | Hinterlegungs-Nummer | Datum der Lebens-fähigkeits-Bescheinigung |
|---|---|---|---|
| pEAP 37 (Escherichia coli DH1 transformiert mit pEAP 37 Plasmid-DNA) | 16. Juni 1987 | DSM 4147 | 19. Juni 1987 |
| pEAP 40 (Escherichia coli DH1 transformiert mit pEAP 40 Plasmid-DNA) | 16. Juni 1987 | DSM 4148 | 19. Juni 1987 |
| pMSV 109 (Escherichia coli JM83Rec A trans-formiert mit pMSV 109 Plasmid-DNA) | 23. Sept. 1987 | NCIB 12547 | 24. Sept. 1987 |

Einschränkungen der Zugänglichkeit besagter Mikroorganismen sind seitens des Hinterlegers nicht verlangt worden.

Beispiel 9: Kultivierung der Agrobacterien-Stämme (RifR) C58 (pTiC58; pEAP 200), (RifR) C58 (pTiC58, pEA 21), C58 (pTiC58, pEA 2) und C58 (pTiC58, pEAP 37), C58 (pTiC58, pEAP 29); C58 (pTiC58, pEAP 40) sowie C58 (pTiC58, pMSV109) und Herstellung der Inokulationslösung

Vor der Inokulation werden die Agrobacterium-Stämme auf YEB Medium [Bacto Rindfleischextrakt 5 g/l, Bacto Hefeextrakt 1 g/l, Peptone 5 g/l, Sucrose 5 g/l, $MgSO_4$ 2mM, pH 7,2], welches zuvor mit 100 µg/ml Rifampicin und 25 µg/ml Kanamycin bzw. 50 µg/ml Nalidixinsäure angereichert und mit 1,5 % Agar verfestigt wurde, ausplattiert. Nach einer Kultivierungsdauer von 48 h bei einer Temperatur von 28°C wird eine einzelen Kolonie zum Animpfen einer Flüssigkultur verwendet. Diese wird in 100 ml Erlenmeyer-Kolben in einem flüssigen YEB-Medium, welches mit Antibiotika in der zuvor angegebenen Konzentration angereichert ist, durchgeführt. Die Kultivierung erfolgt bei einer Temperatur von 28°C auf einer Schüttelmaschine bei einer Geschwindigkeit von 200 r.p.m. Die Kultivierungsdauer beträgt 24 h.

Anschliessend wird eine zweite Subkultivierung in flüssigem Medium mit einem Verdünnungsverhältnis von 1:20 unter ansonsten gleichen Bedingungen durchgeführt. Die Inkubationsdauer beträgt in diesem Fall 20 h.

Diese Massnahmen führen zu einer Populationsdichte an lebenden Agrobakterien von etwa $10^9$/ml.

Die Bakterienzellen werden durch Abzentrifugieren geerntet und anschliessend in einem äquivalenten Volumen einer 10 mM $MgSO_4$-Lösung, die keine Antibiotika enthält, resuspendiert.

Diese Suspension wird im weiteren Verlauf als unverdünnte Stammlösung bezeichnet. Bei der Herstellung von Verdünnungsreihen wird als Verdünnungsmittel ebenfalls 10 mM $MgSO_4$-Lösung verwendet.

Beispiel 10: Sterilisation und Keimung von Mais-Samen

Für die Inokulationsexperimente werden Pflanzen der Varietäten Golden Cross Bantam, B73, North Star und/oder Black Mexican Sweetcorn verwendet, die alle erfolgreich agroinfiziert werden können.

Es werden für die folgenden Experimente in der Regel 3 Tage alte, zuvor sterilisierte, Keimlinge verwendet. Die Sterilisation der Keimlinge umfasst die folgenden Verfahrensschritte:

1. Sterilisation der Samen in einer 0,7 % w/v Calciumhypochlorit-Lösung (250 ml Lösung/100 Samen). Die Durchmischung von Samen und Lösung erfolgt mit Hilfe eines Magnetrührers.

Nach 20 Minuten wird die Sterilisationslösung dekantiert.

2. Es folgt ein 3maliges Waschen des so behandelten Samens mit destilliertem Wasser (250 ml dest. Wasser/100 Samen) für jeweils 30 Minuten.

Die auf diese Weise sterilisierten Samen werden anschliessend in zuvor ebenfalls sterilisierte Keimkammern eingebracht. Dabei handelt es sich um Petrischalen, die jeweils 3 sterile Macherey-Nagel®-Rundfilter mit einem Durchmesser von 8,5 cm sowie ca. 10 ml steriles Wasser enthalten.

In jede dieser Keimkammern werden 20 Samen eingebracht und für ca. 3 Tage bei einer Temperatur von 28°C im Dunkeln inkubiert.

0 267 159

Für die anschliessenden Inokulationsexperimente werden nur Keimlinge verwendet, bei denen der Abstand zwischen Skutellarknoten und der apikalen Koleoptilarspitze 1-2 cm beträgt. Auf alle Fälle muss aber gewährleistet sein, dass der Koleoptilarknoten deutlich identifizierbar ist.

Beispiel 11: Inokulation der Mais-Keimlinge

Für das Einbringen der unter Punkt 3. beschriebenen Inokulationslösung in die Mais-Keimlinge werden Hamilton Injektionsspritzen (A 50 μl oder 100 μl) verwendet, die mit austauschbaren Injektionsnadeln von 0,4 mm Durchmesser ausgestattet sind.

Das Aufziehen der Inokulationslösung in die Injektionsspritze wird in der Weise durchgeführt, dass keine Luftblasen gebildet werden.

11.1. Inokulation 10 Tage alter Maispflanzen

Die Inokulation der bakterienhaltigen Suspension in 10 Tage alte Maispflanzen wird mit verschiedenen Methoden und an verschiedenen Stellen der Pflanze durchgeführt.

1. Aufbringen von 20 μl Bakteriensuspension auf eines der oberen Blätter und Einreiben der Suspension mit Hilfe von Carborundum-Pulver in das Blatt, bis das gesamte Blatt nass erscheint (Position A in Abbildung 1).

2. Injektion von 10 μl der Bakteriensuspension unter Verwendung einer 100 μl Hamilton-Injektionsspritze in den zentralen Teil der Pflanze,

a) genau oberhalb der Ligula des Primärblattes (Position B in Abbildung 1)

b) 1 cm unterhalb der Ligula des Primärblattes (Position C in Abbildung 1)

c) an der Basis der Pflanze in den sog. Wurzelhals, einem meristematischen Gewebe, aus dem sich später Adventivwurzeln entwickeln (Position D in Abbildung 1).

11.2 Inokulation von 3 Tage alten Mais-Keimlingen

Die Inokulation der bakterienhaltigen Suspension in 3 Tage alte Mais-Keimlinge erfolgt durch Injektion in den Keimlinge mit Hilfe einer 100 μl Hamilton Injektionsspritze.

1. Injektion der Bakteriensuspension in den Koleoptilarknoten durch Einführen der Injektionskanüle durch die Koleoptile, ausgehend von der apikalen Koleoptilarspitze bis in den Bereich des Koleoptilarknotens (Position E in Abbildung 2).

2. Injektion der Bakteriensuspension unmittelbar in die Koleoptile, 2 mm unterhalb der apikalen Koleoptilarspitze (Position F in Abbildung 2).

3. Injektion der Bakteriensuspension unmittelbar in die Koleoptile, 2 mm oberhalb des Koleoptilarknotens (Position G in Abbildung 2).

4. Injektion der Bakteriensuspension unmittelbar in den Koleoptilarknoten (Position H in Abbildung 2).

5. Injektion der Bakteriensuspension unmittelbar in die Koleoptile 2 mm unterhalb des Koleoptilarknotens (Position I in Abbildung 2).

6. Injektion der Bakteriensuspension direkt in den Skutellarknoten (Position J in Abbildung 2).

7. Injektion der Bakteriensuspension in den Skutellarknoten durch Einführen der Injektionskanüle durch die Primärwurzel, ausgehend von der Wurzelspitze bis in den Bereich des Skutellarknotens (Position K in Abbildung 2).

11.3 Dekapitieren der Koleoptile im Bereich des Koleoptilarknotens

3 Tage alte Mais-Keimlinge werden an verschiedenen Stellen im Bereich des Koleoptilarknotens dekapitiert (siehe Abbildung 3).

1. unmittelbar auf Höhe des Koleoptilarknotens

2. 1 mm oberhalb des Koleoptilarknotens

3. 2 mm oberhalb des Koleoptilarknotens

4. 5 mm oberhalb des Koleoptilarknotens

Anschliessend werden die dekapitierten Sämlinge in feuchte Erde eingepflanzt und entsprechend den unter Punkt 6. angegebenen Bedingungen kultiviert.

Die eigentlichen Inokulationsexperimente mit Agrobacterium werden an Keimlingen durchgeführt, deren Koleoptilarspitzen 2 mm oberhalb des Koleoptilarknotens präparativ entfernt wurden.

Beispiel 12: Kultivierung der behandelten Mais-Pflanzen und Mais-Keimlinge

Die Mais-Keimlinge werden unmittelbar nach der Inokulationsbehandlung in feuchte Erde eingepflanzt und in gleicher Weise wie die 10 Tage alten Mais-Pflänzchen bei einer Temperatur von 22° ±2°C unter Dauerbeleuchtung mit Weisslicht (Phillips 400 W/G/92/2) bei 3000-5000 lux kultiviert.

Die Pflanzen werden anschliessend täglich auf das Vorhandensein von Symptomen einer Virusinfektion hin untersucht, welche durch das Auftreten von gelben Punkten und/oder Streifen an der Basis neugebildeter Blätter charakterisiert ist.

Beispiel 13: DNA-Extraktion aus infizierten, symptomatischen Mais-pflanzen

Ca. 400 mg (Frischgewicht) junges Blatt-Gewebe wird zunächst in einem Mörser auf Eis homogenisiert, unter Zugabe von 0,5 ml-1,0 ml STEN (15 % Sucrose, 50 mM Tris-HCl, 50 mM $Na_3$ EDTA, 0,25 M NaCl, pH8)

13

und von Sand (~50 mg) zur Unterstützung des Gewebeaufschlusses. Das Homogenisat wird anschliessend in ein Zentrifugen röhrchen (1,5 ml) überführt und 5 Minuten bei einer Temperatur von 4°C in einer Tischzentrifuge bei maximaler Geschwindigkeit zentrifugiert. Der Ueberstand wird verworfen und das Pellet in 0,5 ml eiskaltem SET (15 % Sucrose, 50 mM Na$_3$ EDTA, 50 mM Tris-Hcl, pH 8) unter Rühren zunächst mit einem sterilen Zahnstocher, dann unter kurzzeitiger (5 Sekunden) Verwendung eines Vortex, resuspendiert. Anschliessend werden 10 µl einer 20 % SDS-Lösung sowie 100 µl Proteinase K (20 mg/ml) hinzugegeben, vermischt und anschliessend in dem Röhrchen für 10 Min. auf 68°C erwärmt. Nach der Zugabe von 3 M Natriumacetat (1/10 Volumen) wird das Lysat zweimal mit Phenol/Chloroform (3:1) extrahiert. Die DNA wird dann durch Zugabe von 2 Volumenteilen Ethanol prezipitiert und über Nacht bei -20°C aufbewahrt. Durch Zentrifugation (10 Min.) in einer Tischzentrifuge bei maximaler Geschwindigkeit erhält man ein DNA-haltiges Pellet, das anschliessend in 40 µl TE-Puffer (40 mM Tris-HCl, 1 mM Na$_3$ EDTA, pH 8) gelöst wird. Aliquots dieser DNA-Lösung werden für die "Southern blot"-Experimente (36) Southern EM, J.Mol.Biol., 98: 503-517, 1975) verwendet.

Beispiel 14: "Southern blot"-Analyse

Die extrahierte DNA wird zunächst mit Restriktionsenzymen behandelt, anschliessend einer Elektrophorese in 1 %-igem Agarose-Gel unterworfen, auf eine Nitrozellulosemembran [22] Southern, E.M., J.Mol.Biol. 98, 503-517 (1975)] transferiert und mit der nachzuweisenden DNA, welche zuvor einer Nick Translation [23] Rigby, W.J., Dieckmann, M., Rhodes, C. und P. Berg. J.Mol.Biol. 113, 237-251)] unterworfen wurde (DNA-spezifische Aktivitäten von 5 x 10$^8$ bis 10 x 10$^8$ c.p.m./µg) hybridisiert. Die Filter werden dreimal je eine Stunde lang mit einer wässrigen Lösung von 0.03 M Natriumcitrat und 0.3 M Natriumchlorid bei 65°C gewaschen. Die hybridisierte DNA wird durch Schwärzung eines Röntgenfilms während 24 bis 48 Stunden sichtbar gemacht.

Ergebnisse:

A) Inokulation 10 Tage alter Mais-Pflanzen

Tabelle 1 gibt die Ergebnisse von Inokulationsexperimenten an 10 Tage alten Mais-Pflanzen wieder, die unter Punkt 10.1. Beschrieben sind. Die Inokulation erfolgt unter Verwendung von pEAP 37 DNA.

## Tabelle 1

| Inokulations-ort | Zahl der Pflanzen mit Symptomen/ Zahl der inokulierten Pflanzen | | | |
|---|---|---|---|---|
| | pEAP 37 | pMSV 109 | pEAP 200 | pEAP 25 |
| A | 0/46 (<2 %) | — | — | — |
| B | 0/44 (<2 %) | — | — | — |
| C | 3/46 (6,5 %) | — | + | + |
| D | 42/68 (62 %) | 26/65 (40 %) | ++ | ++ |

Die Ergebnisse der Tabelle 1 machen deutlich, dass der bevorzugte Applikationsort an der Pflanze im Bereich des Wurzelhalses lokalisiert ist, wo 62 % bzw. 40 % der behandelten Pflanzen Infektionssymptome zeigen, während die Zahl der infizierten Pflanzen bei Inokulation an den übrigen Inokulationspunkten der Pflanze (A, B, C) 0 oder vernachlässigbar gering ist.

B) Inokulation von 3 Tage alten Mais-Keimlingen

Tabelle 2 gibt die Ergebnisse von Inokulationsexperimenten an 3 Tage alten Mais-Keimlingen wieder, die unter Punkt 10.2. beschrieben sind. Die Inokulation erfolgt unter Verwendung von pEAP 37 und pEAP 40 DNA.

Tabelle 2

| Inokulationsort | Zahl der Pflanzen mit Symptomen/ Zahl der inokulierten Pflanzen | |
| --- | --- | --- |
| | pEAP 37 | pEAP 40 |
| E | 21/27 (78 %) | ------ |
| F | 0/20 (<5 %) | ------ |
| G | 3/19 (16 %) | ------ |
| H | 25/30 (83 %) | 51/58 (88 %) |
| I | 8/51 (16 %) | ------ |
| J | 1/20 ( 5 %) | ------ |
| K | 2/12 (17 %) | ------ |

Wie die Ergebnisse der Tabelle 2 zeigen, befindet sich der bevorzugte Applikationsort beim Mais-Keimling im Bereich des Koleoptilarknotens, wobei die direkte und indirekte Applikation der Bakteriensuspension unmittelbar in den Koleoptilarknoten mit 83 % und 88 % bzw. 78 % infizierter Pflanzen eindeutig bevorzugt ist, gegenüber allen anderen untersuchten Applikationsorten. Dabei spielt es offentsichtlich keine Rolle, ob die Injektion der Suspension direkt seitlich in den Koleoptilarknoten erfolgt oder über den Umweg durch die Koleoptile.

C) Dekaptitation 3 Tage alter Mais-Keimlinge
Tabelle 3 gibt die Anzahl der überlebenden Keimlinge 2 Wochen nach Dekaptitation der Koleoptile an verschiedenen Stellen im Bereich des Koleoptilarknotens wieder.

Tabelle 3

| Dekaptitations- stelle | Zahl der überlebenden Keimlinge/ Zahl der dekapitierten Keimlinge |
| --- | --- |
| 1 | 0/7 |
| 2 | 5/8 |
| 3 | 8/8 |
| 4 | 8/8 |

Es zeigt sich, dass die Plumula bis zu 2 mm oberhalb des Koleoptilarknotens entfernt werden kann, ohne dass eine Beeinträchtigung der Lebensfähigkeit der so behandelten Keimlinge beobachtet werden kann. Auch das Entfernen der Plumula nur 1 mm oberhalb des Koleoptilarknotens führt noch in ca. 60 % der Fälle zu vollständig lebensfähigen Pflänzchen.

Tabelle 4 gibt die Ergebnisse von Inokulationsexperimenten an 2 mm oberhalb des Koleoptilarknotens dekapitierten Keimlingen wieder. Die Inokulation erfolgt unter Verwendung von pEAP 37 DNA.

Tabelle 4

| Inokulationsort | Zahl der Pflanzen mit Symptomen/ Zahl der inokulierten Pflanzen |
|---|---|
| L | 48/49 (98 %) |
| M | 14/44 (32 %) |

Die Ergebnisse der Tabelle 4 machen deutlich, das die Position L am dekapitierten Keimling, d.h. der meristematischen Gewebebereiche, eindeutig bevorzugt ist gegenüber der Position M, die die peripheren Gewebeabschnitte abdeckt.

D) Verdünnungsexperimente

Die unter Punkt 9 beschriebene Bakteriensuspension wird in YEB Medium ohne Antibiotikazusatz verdünnt und in den unten angegebenen Konzentrationen in den Koleoptilarknoten appliziert.

| Verdünnung | Geschätzte in der Inokulationsstelle verbleibende Bakterienzahl | Anzahl der Pflanzen mit Symptomen/ Anzahl der inokulierten Pflanzen |
|---|---|---|
| unverdünnt | $2 \times 10^6$ | 84/102 (82 %) |
| $10^{-1}$ | $2 \times 10^5$ | 42/55 (76 %) |
| $10^{-2}$ | $2 \times 10^4$ | 34/54 (62 %) |
| $10^{-3}$ | $2 \times 10^3$ | 19/56 (34 %) |
| $10^{-4}$ | 0 | 0/10 (<10 %) |
| $10^{-5}$ | 0 | 0/10 (<10 %) |

Geht man davon aus, dass die Kopienzahl des binären Vektors, der die MSV-Sequenzen enthält, ungefähr 10 beträgt und dass die Bakterien sich in der Inokulationsstelle nicht weiter vermehren, dann enthalten $10^4$ Bakterien ca. 400 fg ($4 \times 10^{13}$ g) MSV DNA.

Das bedeutet, dass Agrobacterium seine DNA mit der einer vergleichbaren Effizienz auf Mais überträgt, wie dies bei dikotylen Wirtspflanzen der Fall ist.

E) Agrobacterium-Wirtsbereich

Neben Mais konnten noch weitere Vertreter aus der Klasse Gramineae ermittelt werden, die einer Infektion mit Agrobacterium zugänglich sind.

Die Ergebnisse von Inokulationsexperimenten mit diesen Gramineen-Spezies sind in Tabelle 5 wiedergegeben:

Tabelle 5

| Gramineen Spezies | Zahl der Pflanzen mit Symptomen/ Zahl der inokulierten Pflanzen |
|---|---|
| Gerste (Maris Otter) | 1/15 (6 %) |
| Weizen (Maris Butler) | 1/40 (2 %) |
| Weizen (Normal) | 1/25 (4 %) |
| Flughafer (Saladin) | 1/25 (4 %) |
| Panicun milaceum | 3/8 (35 %) |
| Digitaria Sanguinalis | 2/10 (20 %) |
| Lolium temulentum | 1/25 (4 %) |

Einige der weniger effektiven Ergebnisse sind möglicherweise auf technische Schwierigkeiten im Verlaufe der Inokulation zurückzuführen, da die Pflanzen z.T. sehr klein sind und daher nur geringe Stengeldurchmesser aufweisen, was eine zielgerichtete Injektion der Inokulationslösung schwierig macht.

Dessen ungeachtet zeigen die vorliegenden Ergebnisse, dass neben Mais eine Reihe weiterer Vertreter aus der Gruppe der Gramineen durch Agrobacterium transformierbar sind.

F) Agrobacterium-Stämme

Neben dem in den Inokulationsexperimenten mit Mais routinemässig verwendeten Agrobacterium tumefaciens-Stamm C58, wurden noch weitere A. tumefaciens und A. rhizogenes-Stämme getestet. Dabei konnte bei Verwendung der folgenden in Tabelle 6 aufgelisteten Agrobacterium Stämme ebenfalls eine Uebertragung der MSV-DNA auf Mais nachgewiesen werden:

Tabelle 6:

| Agrobacterium Stamm | Zahl der Pflanzen mit Symptomen Zahl der inokulierten Pflanzen | |
|---|---|---|
| | pMSV 109 *1 | pEAP 37 *2 |
| A. tumefaciens | | |
| T 37 | 3/6 (50 %) | 6/6 (100 %) |
| LBA 4301 (pTiC58) | 21/23 (91 %) | 15/21 (71 %) |
| A 6 | 0/8 (<1 %) | 2/37 (5 %) |
| A. rhizogenes | | |
| R 1000 | 17/22 (81 %) | —— |
| LBA 9402 | 15/20 (75 %) | —— |
| 2626 | 7/12 (51 %) | —— |

*1 die Inokulationsexperimente mit pMSV 109 wurden an 10 Tage alten Maispflanzen durchgeführt

*2 die Inokulationsexperimente mit pEAP 37 wurden an 3 Tage alten Maiskeimlingen durchgeführt

Literaturverzeichnis
1) DeCleene M, Phytopath. Z., 113, pp 81-89, (1985)
2) Hernalsteens JP et al., EMBO J 3, pp 3039-3041, (1984)

3) Hooykaas-Van Slogteren GMS et al., Nature 311, pp 763-764,

4) Graves AFC and Goldman SL, J. Bacteriol., 169(4): 1745-1746, 1987

5) Hain R et al., Mol. Gen. Genet., 199: 161-168, 1985

6) Paszkowski J et al., EMBO J, 3: 2717-2722, 1984

7) Potrykus I et al., (1985a) "Direct gene transfer to protoplasts: an efficient and generally applicable method for stable alterations of plant genoms", In: Freeling M. (ed.), Plant Genetics Alan R. Liss Inc., New York, pp. 181-199

8) Lörz H et al., Mol. Gen. Genet., 119: 178-182, 1985

9) Fromm M et al., Proc. Natl. Acad. Sci. USA, 82: 5824-5828, 1985

10) Steinbiss HH and Stabel P, Protoplasma 116: 223-227; 1983

11) Morikawa H and Yamaday, Plant Cell Physiol., 26: 229-236, 1985

12) Stachel et al., Nature, 318: 624-629, 1985

13) Jacob F et al., Cold Spring Harbor Symp. 28, 329 (1963)

14) Cell Culture and Somatic Cell Genetics of Plants, Vol. 1, ed. IK Vasil, Academic Press, 1984

15) Pareddy DR, et al., Planta 170; pp 141-143, Leemans, J. et al., Gene 19, pp 361-364, (1982)

16) Mullineaux PM et al., EMBO J, 3(13): 3063-3068, 1984

17) Kegami M et al., Proc. Natl. Acad. Sci. USA, 78, pp 4102 (1981)

18) Donson J et al., EMBO J, 3(13): 3069-3073, 1984

19) Vieira T and Messing T, Gene, 19: 259-268, 1982

20) Maniatis T et al., Molecular Cloning, a Laboratory Manual, Cold Spring Harbor, (1982)

21) An G et al., EMBO J: 277-284, 1985

22) Hanahan D and Meselson M, Gene, 10: 63-67, 1980

23) Rogers SG et al., Methods in Enzymology, 118: 630-633, 1986

24) Dhaese P et al., Nucl. Acids Res., 7: 1837-1849, 1979

25) Holsters et al., Plasmid, 3: 2,12, 1980

26) Bevan M., Nucl. Acids Res., 12: 204-207, 1984

27) Hohn, B. et Collins, J., Gene 11, pp 291-298 (1980)

28) Grindley N.D.F., et al., Proc. Natl. Acad. Sci. USA, 77, pp 7176-7180, (1980)

29) Grimsley NH, et al., Nature,325(8), pp 177-179, (1987)

30) Chang ACY et Cohen SN, J. Bacteriol. 134, pp. 1141-1156, (1978)

31) Mead DA, et al., Protein Engineering 1, pp 67-74, (1986)

32) Rothstein SJ, et al., Gene, 53, pp 153-161, (1987)

33) Hepburn, et al. ...........(1985)

34) Ditta G, et al., Proc. Natl. Acad. Sci., USA, 77(12), pp 7347-7351, (1980)

35) van Haute E et al., EMBO J. 2, No. 3 pp 411-417, (1983)

36) Southern EM, J. Mol. Biol., 98: 503-517, (1975)

**Patentansprüche**

1. Verfahren zur Einschleusung von genetischem Material in monokotyle Pflanzen oder lebensfähige Teile davon, dadurch gekennzeichnet, dass man einen Transfer-Mikroorganismus, der das genetische Material in einer transportierbaren Form enthält, und der in der Lage ist besagtes genetisches Material in monokotyle Pflanzen oder lebensfähige Teile davon einzuschleusen durch Anwendung geeigneter Kultur und Applikationsmethoden, die eine Induktion der Virulenz-Genfunktionen des Transfer-Mikroorganismus ermöglichen, für eine Infektion von Monokotyledonen einsetzbar macht und mit besagtem Transfer-Mikroorganismus monokotyle Pflanzen oder lebensfähige Teile davon infiziert.

2. Verfahren gemäss Anspruch 1, dadurch gekennzeichnet, dass man besagten Transfer-Mikroorganismen in an sich bekannten Kulturmedien heranzieht, wobei gegebenenfalls ein oder mehrere Subkultivierungsschritte durchgeführt werden, die Transfer-Mikroorganismen abzentrifugiert und anschliessend in einem geeigneten Medium resuspendiert und man die so erhaltene Inokulationslösung in eine Wachstumszone einer monokotylen Pflanze einbringt.

3. Verfahren gemäss einem der Ansprüche 1 oder 2, dadurch gekennzeichnet, dass man besagten Transfer-Mikroorganismus in einer Schüttelkultur über einen Zeitraum von 30 bis 60 Stunden (h), bei einer Inkubationstemperatur von 15° bis 40°C, in einem für die Kultivierung von Transfer-Mikroorganismen geeigneten Medium heranzieht und anschliessend gegebenenfalls einen oder mehrere Subkultivierungsschritte durchführt, wobei jeder einzelne dieser Subkultivierungsschritte über einen Zeitraum von 15 bis 30 Stunden erfolgt und bei einer Temperatur von 15° bis 40°C durchgeführt wird.

4. Verfahren gemäss Anspruch 3, dadurch gekennzeichnet, dass der Inkubationszeitraum für die Kultivierung sowie die gegebenenfalls erforderlichen Subkultivierungen des Transfer-Mikroorganismus 40 bis 50 Stunden beträgt.

5. Verfahren gemäss Anspruch 3, dadurch gekennzeichnet, dass die Inkubationstemperatur für die Kultivierung sowie die gegebenenfalls erforderlichen Subkultivierungen des Transfer-Mikroorganismus 24° bis 29°C beträgt.

6. Verfahren gemäss einem der Ansprüche 1 bis 3, dadurch gekennzeichnet, dass man ein mit Agarose oder Alginat oder jedem beliebigen anderen geeigneten Verfestigungsmittel verfestigtes Kulturmedium verwendet.

7. Verfahren gemäss Anspruch 1, dadurch gekennzeichnet, dass man besagte Transfer-Mikroorganismen in Form einer Mikroorganismensuspension in die meristematischen Gewebe-Regionen der Pflanze oder lebensfähige Teile davon inokuliert.

8. Verfahren gemäss Anspruch 7, dadurch gekennzeichnet, dass man besagte Mikroorganismensuspension mehrfach inokuliert.

9. Verfahren gemäss Anspruch 1, dadurch gekennzeichnet, dass man durch Wahl des Inokulationszeitpunktes - im Hinblick auf das Entwicklungs-und Differenzierungsstadium der Pflanze - sowie des Inokulationsortes auf der Pflanze in der Weise aufeinander abstimmt, dass eine signifikante Erhöhung der Transformationshäufigkeit resultiert.

10. Verfahren gemäss Anspruch 9, dadurch gekennzeichnet, dass man die Inokulation der transformierenden Mikroorganismensuspension an einer Pflanze vornimmt, die sich in einem Entwicklungsstadium befindet, welches zwischen dem Beginn der Entwicklung des pflanzlichen Embryos und dem Beginn der Blütenbildung liegt.

11. Verfahren gemäss Anspruch 10, dadurch gekennzeichnet, dass sich besagte Empfängerpflanze in einem Entwicklungsstadium zwischen der Samenkeimung und dem Erreichen des 4-Blattstadiums befindet.

12. Verfahren gemäss Anspruch 11, dadurch gekennzeichnet, dass sich die Pflanze im 1. bis 3. Tag der Keimungsphase befindet, wobei der Abstand zwischen dem Skutellarknoten und der apikalen Koleoptilarspitze ca. 1-3 cm beträgt.

13. Verfahren gemäss Anspruch 7, dadurch gekennzeichnet, dass die Inokulation der transformierenden Mikroorganismensuspension durch Injektion in die meristematischen Gewebe-Regionen der Pflanze erfolgt.

14. Verfahren gemäss Anspruch 13, dadurch gekennzeichnet, dass die Injektion der transformierenden Mikroorganismensuspension beim bereits in Wurzel, Stengel und Blättern differenzierten Pflänzchen im Bereich des Wurzelhalses erfolgt.

15. Verfahren gemäss Anspruch 13, dadurch gekennzeichnet, dass die Injektion der transformierenden Mikroorganismensuspension beim Keimling in die unmittelbare Nähe des Koleoptilarknotens erfolgt.

16. Verfahren gemäss Anspruch 15, dadurch gekennzeichnet, dass die Injektion der transformierenden Mikroorganismensuspension direkt in den Koleoptilarknoten oder innerhalb eines Bereichs von ca. 2 mm um den Koleoptilarknoten erfolgt.

17. Verfahren gemäss Anspruch 13, dadurch gekennzeichnet, dass die Injektion der transformierenden Mikroorganismensuspension nach Dekapitieren der Koleoptilarspitze unmittelbar in die meristematischen Gewebebereiche der Koleoptile erfolgt.

18. Verfahren gemäss Anspruch 17, dadurch gekennzeichnet, dass die Dekapitation der Koleoptilarspitze in einem Bereich, der sich 1-5 mm oberhalb des Koleoptilarknotens befindet, erfolgt.

19. Verfahren gemäss Anspruch 1, dadurch gekennzeichnet, dass es sich bei besagtem genetischem Material um virale DNA handelt, die gegebenenfalls eingebaute Cargo-DNA enthalten kann.

20. Verfahren gemäss Anspruch 19 zur Einschleusung viraler DNA, die gegebenenfalls Cargo-DNA eingebaut enthält, in Pflanzen aus der Klasse Monocotyledoneae dadurch gekennzeichnet, dass man

a) in ein T-Replikon virale DNA, welche gegebenenfalls Cargo-DNA eingebaut enthält, in Nachbarschaft zu einer oder mehreren T-DNA-Grenz-Sequenzen integriert, wobei die Entfernung zwischen viraler DNA und T-DNA-Grenz-Sequenz(en) so gewählt ist, dass die Uebertragung der viralen DNA einschliesslich gegebenenfalls vorhandener Cargo-DNA in pflanzliches Material erfolgt.

b) anschliessend die Aufnahme des T-Replikons in einen geeigneten Transfer-Mikroorganismus veranlasst, wobei das Replikon in den Transfer-, Mikroorganismus gelangt,

c) mit dem gemäss b) modifizierten Transfer-Mikroorganismus Pflanzen aus der Klasse Monocotyledoneae infiziert.

21. Verfahren gemäss Anspruch 19, dadurch gekennzeichnet, dass man

a) virale DNA oder ihre Aequivalente aus infiziertem pflanzlichem Material isoliert und mit Hilfe geeigneter Vektoren in einem Wirtsorganismus kloniert;

b) die klonierte virale DNA oder Teile davon sowie eventuell darin eingebaute Cargo-DNA zum Aufbau eines bakteriellen Plasmids ( =BaP) verwendet, welches mehr als ein virales Genom oder Teile viraler Genome sowie eventuell darin eingebaute Cargo-DNA enthält, welche sich in Nachbarschaft zu einer oder mehreren T-DNA-Grenz-Sequenzen befinden, wobei die Entfernung zwischen viraler DNA und T-DNA-Grenz-Sequenzen so gewählt ist, dass die Uebertragung der viralen DNA einschliesslich der gegebenenfalls darin eingebauten Cargo-DNA in pflanzliches Material erfolgt;

c) zum Aufbau eines für Pflanzen verwendbaren Vektorsystem das Plasmid BaP in einen geeigneten Transfer-Mikroorganismus überführt,

d) Pflanzen aus der Klasse Monocotyledoneae oder lebensfähige Teile davon mit dem so modifizierten Vektorsystem infiziert.

22. Verfahren gemäss einem der Ansprüche 19 bis 21, dadurch gekennzeichnet, dass man als virale DNA doppelsträngige DNA-Formen von Einzelstrang-DNA-Viren verwendet.

23. Verfahren gemäss Anspruch 22, dadurch gekennzeichnet, dass man als virale DNA solche von Gemini-Viren verwendet.

24. Verfahren gemäss Anspruch 23, dadurch gekennzeichnet, dass man als virale DNA solche von Maize Streak Virus (MSV), Bean Golden Mosaik Virus (BGMV), Chloris Striate Mosaic Virus (CSMV), Cassava Latent Virus (CLV), Curly Top Virus (CTV), Tomato Golden Mosaic Virus (TGMV) oder Wheat Dwarf Virus (WDV) verwendet.

25. Verfahren gemäss einem der Ansprüche 19 bis 21, dadurch gekennzeichnet, dass man als virale DNA natürliche Virus-DNA verwendet.

26. Verfahren gemäss Anspruch 25, dadurch gekennzeichnet, dass man als virale DNA solche von Cauliflower-Mosaik-Virus verwendet.

27. Verfahren gemäss einem der Ansprüche 19 bis 21, dadurch gekennzeichnet, dass man als virale DNA cDNA-Kopien von Virus-RNA verwendet.

28. Verfahren gemäss einem der Ansprüche 19 bis 21, dadurch gekennzeichnet, dass man als virale DNA cDNA-Kopien von Viroid-RNA verwendet.

29. Verfahren gemäss einem der Ansprüche 19 bis 28, dadurch gekennzeichnet, dass man als virale DNA solche von lethalen oder lebensfähige Mutanten von Viren verwendet.

30. Verfahren gemäss Anspruch 19, dadurch gekennzeichnet, dass man als virale DNA unter der Kontrolle viraler Replikationssignale stehende, klonierte DNA verwendet.

31. Verfahren gemäss Anspruch 19, dadurch gekennzeichnet, dass man als virale DNA unter der Kontrolle viraler Expressionssignale stehende klonierte DNA verwendet.

32. Verfahren gemäss Anspruch 19, dadurch gekennzeichnet, dass man als virale DNA unter der Kontrolle viraler Replikations- und Expressionssignale stehende klonierte DNA verwendet.

33. Verfahren gemäss Anspruch 19, dadurch gekennzeichnet, dass man als virale DNA unter der Kontrolle eukaryontischer Replikations- und Expressionssignale stehende klonierte DNA verwendet.

34. Verfahren gemäss Anspruch 19, dadurch gekennzeichnet, dass man als virale DNA Teile von Virus-DNA verwendet.

35. Verfahren gemäss Anspruch 19, dadurch gekennzeichnet, dass man die virale DNA in Tandem-Form verwendet.

36. Verfahren gemäss einem der Ansprüche 19 bis 34, dadurch gekennzeichnet, dass man die virale DNA oder Aequivalente davon in Tandem-Form verwendet.

37. Verfahren gemäss einem der Ansprüche 19 bis 36, dadurch gekennzeichnet, dass man virale DNA mit eingebauter Cargo-DNA verwendet.

38. Verfahren gemäss einem der Ansprüche 19 bis 36, dadurch gekennzeichnet, dass man virale DNA oder Aequivalente davon mit eingebauter Cargo-DNA verwendet.

39. Verfahren gemäss einem der Ansprüche 20 oder 21, dadurch gekennzeichnet, dass man ein bakterielles T-Replikon verwendet.

40. Verfahren gemäss Anspruch 39, dadurch gekennzeichnet, dass man ein T-Replikon von einem Bakterium der Gattung Agrobacterium verwendet.

41. Verfahren gemäss einem der Ansprüche 39 oder 40, dadurch gekennzeichnet, dass man als T-Replikon ein Ti-Plasmid oder ein Ri-Plasmid aus einem Bakterium der Gattung Agrobacterium verwendet.

42. Verfahren gemäss Anspruch 1, dadurch gekennzeichnet, dass man als Mikroorganismus, welcher ein T-Replikon gemäss einem der Ansprüche 39 bis 41 beherbergt, ein Bakterium verwendet.

43. Verfahren gemäss Anspruch 42, dadurch gekennzeichnet, dass man als Mikroorganismus, welcher das T-Replikon beherbergt, ein Bodenbakterium verwendet.

44. Verfahren gemäss Anspruch 43, dadurch gekennzeichnet, dass man als Mikroorganismus, welcher das T-Replikon beherbergt, ein Bakterium der Gattung Agrobacterium verwendet.

45. Verfahren gemäss Anspruch 38, dadurch gekennzeichnet, dass besagte Cargo-DNA entweder aus genomischer DNA aus cDNA oder aus synthetischer DNA besteht.

46. Verfahren gemäss Anspruch 38, dadurch gekennzeichnet, dass besagte Cargo-DNA sowohl aus genomischer als auch aus cDNA und/oder synthetischer DNA zusammengesetzt ist.

47. Verfahren gemäss Anspruch 38, dadurch gekennzeichnet, dass besagte Cargo-DNA aus Genfragementen von mehreren Organismen, die verschiedenen Gattungen angehören, zusammengesetzt ist.

48. Verfahren gemäss Anspruch 38, dadurch gekennzeichnet, dass besagte Cargo-DNA aus Genfragmenten von mehr als einem Stamm, einer Varietät oder einer Spezies des gleichen Organismus zusammengesetzt ist.

49. Verfahren gemäss Anspruch 38, dadurch gekennzeichnet, dass besagte Cargo-DNA aus Anteilen von mehr als einem Gen derselben Organismen zusammengesetzt ist.

50. Verfahren nach Anspruch 1, dadurch gekennzeichnet, dass man als lebensfähige Teile von monokotylen Pflanzen pflanzliche Gewebekulturen oder Zellkulturzellen verwendet.

51. Verfahren nach Anspruch 50, dadurch gekennzeichnet, dass man als Pflanzen oder lebensfähige Teile davon Pflanzen aus der Familie der Alliaceae, Amaryllidaceae, Asparagaceae, Bromeliaceae, Gramineae, Liliaceae, Musaceae, Orchidaceae oder Palmae verwendet.

52. Verfahren nach Anspruch 51, dadurch gekennzeichnet, dass man als Pflanzen oder lebensfähige Teile dieser Pflanzen solche aus der Familie der Gramineae verwendet.

53. Verfahren nach Anspruch 52, dadurch gekennzeichnet, dass man als Pflanzen oder lebensfähige Teile von Pflanzen Mais, Reis, Weizen, Gerste, Roggen, Hafer oder Hirse verwendet.

54. Verfahren nach Anspruch 50, dadurch gekennzeichnet, dass man als Pflanzen oder lebensfähige Teile von Pflanzen, solche aus der Gattungen Allium, Avena, Hordeum, Oryzae, Panicum, Saccharum, Secale, Setaria, Sorghum, Triticum, Zea, Musa, Cocos, Phoenix, Elaeis oder Teile dieser Pflanzen verwendet.

55. Verfahren nach Anspruch 1, dadurch gekennzeichnet, dass man Protoplasten zusammen mit dem Transfer-Mikroorganismus inkubiert.

56. Transfer-Mikroorganismus oder relevante Teile davon, die in dem Verfahren gemäss Anspruch 1 eingesetzt werden können.

57. Plasmid pEAP 37 oder pEAP 40 oder Transfer-Mikroorganismus enthaltend eines dieser Plasmide.

58. Das Plasmid pMSV 109 oder Transfer-Mikroorganismus enthaltend dieses Plasmid.

59. Der transformierte Escherichia coli-Stamm DH1 (pEAP 37), von dem eine Probe unter der Hinterlegungs-Nummer DSM 4147 hinterlegt worden ist, sowie alle Abkömmlinge und Mutanten davon, die noch die charakteristischen Eigenschaften des transformierten Stammes besitzen.

60. Der transformierte Escherichia coli-Stamm DH1 (pEAp 40), von dem eine Probe unter der Hinterlegungs-Nummer DSM 4148 hinterlegt worden ist, sowie alle Abkömmlinge und Mutanten davon, die noch die charakteristischen Eigenschaften des transformierten Stammes besitzen.

61. Der transformierte Escherichia coli-Stamm JM83Rec A, von dem eine Probe unter der Hinterlegungsnummer NCIB 12547 hinterlegt worden ist, sowie alle Abkömmlinge und Mutanten davon, die noch die charakteristischen Eigenschaften des transformierten Stammes besitzen.

62. Monokotyle Pflanzen und deren Nachkommen, hergestellt gemäss einem der Verfahren der Ansprüche 1 bis 49, dadurch gekennzeichnet, dass ein Grossteil der somatischen Zellen und/oder der Keimzellen besagter Pflanzen sowie deren Nachkommen transformiert sind.

63. Samen von Pflanzen und deren Nachkommen gemäss Anspruch 62.

64. Monokotyle Pflanzen oder lebensfähige Teile von monokotylen Pflanzen, welche gemäss dem in dem Ansprüchen 1 bis 49 beschriebenen Verfahren transformiert worden sind.

65. Transformierte Teile von monokotylen Pflanzen gemäss Anspruch 64, dadurch gekennzeichnet, dass es sich um Samen, Pollen, Eizellen, Zygoten, Embryonen oder anderes aus transformierten Keimbahnzellen hervorgegangenes Vermehrungsgut handelt.

66. Eine vollständig durchtransformierte Pflanze, welches aus lebensfähigen Teile von monokotylen Pflanzen gemäss Anspruch 50 oder 65 regeneriert worden ist.

67. Die aus dem in dem Ansprüchen 1 bis 49 beanspruchten Verfahren resultierenden transformierten Protoplasten, Pflanzenzellen, Zellaggregate, Pflanzen und Samen sowie deren Nachkommen, die die durch die Transformation erzeugte neue Eigenschaften aufweisen.

68. Alle Kreuzungs- und Fusionsprodukte mit dem in den Ansprüchen 62 bis 67 definierten transformierten Pflanzenmaterial, die die durch die Transformation erzeugten neuen Eigenschaften aufweisen.

69. Verwendung des Transfer-Mikroorganismus gemäss Anspruch 1 im Pflanzenschutz zur "Immunisierung" von Pflanzen gegen unerwünschten Virusbefall.